# EUROPEAN PATENT SPECIFICATION

(11) **EP 3 914 611 B1**
(45) Date of publication and mention of the grant of the patent: **21.08.2024**
(21) Application number: 20701871.4
(22) Date of filing: 20.01.2020
(51) Int. Cl.: C07K 14/725, A61K 35/17

(54) **RECEPTORS PROVIDING TARGETED COSTIMULATION FOR ADOPTIVE CELL THERAPY**
REZEPTOREN MIT GEZIELTER COSTIMULIERUNG FÜR ADOPTIVE ZELLTHERAPIE
RÉCEPTEURS FOURNISSANT UNE COSTIMULATION CIBLÉE POUR UNE THÉRAPIE CELLULAIRE ADOPTIVE

(30) Priority: 22.01.2019 GB 201900858; 20.12.2019 US 201962951770 P
(43) Date of publication of application: 01.12.2021
(73) Proprietor: INSTIL BIO (UK) LIMITED, Manchester M13 9XX (GB)
(72) Inventor: BRIDGEMAN, John, Manchester Greater Manchester M13 9XX (GB); HAWKINS, Robert, Manchester Greater Manchester M13 9XX (GB)
(74) Representative: Murgitroyd & Company
(86) International application number: PCT/GB2020/050120
(87) International publication number: WO 2020/152451

(56) References cited:
- WO-A1-2016/141357
- E. LANITIS ET AL: "Chimeric Antigen Receptor T Cells with Dissociated Signaling Domains Exhibit Focused Antitumor Activity with Reduced Potential for Toxicity In Vivo", CANCER IMMUNOLOGY RESEARCH, vol. 1, no. 1, 7 April 2013 (2013-04-07), pages 43 - 53, XP055170367, ISSN: 2326-6066, DOI: 10.1158/2326-6066.CIR-13-0008
- ALVAREZ-VALLINA L ET AL: "ANTIGEN-SPECIFIC TARGETING OF CD28-MEDIATED T CELL CO-STIMULATION USING CHIMERIC SINGLE-CHAIN ANTIBODY VARIABLE FRAGMENT-CD28 RECEPTORS", EUROPEAN JOURNAL OF IMMUNOLOGY, WILEY VCH, WEINHEIM, vol. 26, no. 10, 1 October 1996 (1996-10-01), pages 2304 - 2309, XP000646504, ISSN: 0014-2980, DOI: 10.1002/EJI.1830261006
- KEYU LI ET AL: "Chimeric Antigen Receptor-Engineered T Cells for Liver Cancers, Progress and Obstacles", TUMOR BIOLOGY, vol. 39, no. 3, 28 March 2017 (2017-03-28), pages 1 - 8, XP055571345, DOI: 10.1177/1010428317692229

## Description

### SEQUENCE STATEMENT

The instant application contains a Sequence Listing, which has been submitted electronically and is hereby incorporated by reference in its entirety.

### FIELD OF THE INVENTION

The present invention relates to a cell comprising a chimeric costimulatory antigen receptor (CoStAR) useful in adoptive cell therapy (ACT). The CoStAR can act as a modulator of cellular activity enhancing responses to defined antigens. The present invention also provides CoStAR proteins, nucleic acids encoding the CoStAR and therapeutic uses thereof.

### BACKGROUND OF THE INVENTION

Adoptive cell therapy (ACT) using autologous T-cells to mediate cancer regression has shown much promise in early clinical trials. Several general approaches have been taken such as the use of naturally occurring tumour reactive or tumour infiltrating lymphocytes (TILs) expanded ex vivo. Additionally, T-cells may be genetically modified to retarget them towards defined tumour antigens. This can be done via the gene transfer of peptide (p)-major histocompatibility complex (MHC) specific T-cell Receptors (TCRs) or synthetic fusions between tumour specific single chain antibody fragment (scFv) and T-cell signalling domains (e.g. CD3ζ), the latter being termed chimeric antigen receptors (CARs).

TIL and TCR transfer has proven particularly good when targeting Melanoma (Rosenberg et al. 2011; Morgan 2006), whereas CAR therapy has shown much promise in the treatment of certain B-cell malignancies (Grupp et al. 2013).

The success of CAR therapy in leukaemia has been partly attributed to the incorporation of costimulatory domains (e.g. CD28 or CD137) into the CAR construct, signals from which synergise with the signal provided by CD3ζ to enhance anti-tumour activity. The basis of this observation relates to the classical signal1/signal 2 paradigm of T-cell activation. Here signal 1, provided by the TCR complex, synergises with signal 2 provided by costimulatory receptors such as CD28, CD137 or CD134 to permit the cells to undergo clonal expansion, IL2 production and long term survival without the activation induced cell death (AICD) associated with signal 1 alone. Furthermore the involvement of signal 2 enhances the signal generated through signal 1 allowing the cells to respond better to low avidity interactions such as those encountered during anti-tumour responses.

Targeted costimulation will have beneficial effects for non-CAR-based T cell therapies. For example, incorporating costimulatory domains into a chimeric TCR has been shown to enhance responses of T-cells towards pMHC (Govers 2014). While tumor infiltrating lymphocytes (TILs) utilise their endogenous TCRs to mediate tumour recognition, it has not been possible to engineer the endogenous TCR. Thus TIL are subject to substantial limitations as tumour cells express very few costimulatory ligands. The ability to induce targeted costimulation of TIL, or indeed any other adoptive T-cell therapy product, would be beneficial.

### SUMMARY OF THE INVENTION

The CoStARs and cells comprising or expressing the CoStARs as described herein are beneficial for CAR and non-CAR based T-cell therapies alike. The present invention uses cells according to claim 10 that express a novel chimeric costimulatory receptor according to claim 1 to provide a costimulatory signal to T-cells upon engagement with a defined disease-associated, for example tumour-associated, antigen. There have been several reports in which split signal 1 and signal 2 have been used to drive antigen specific responses in engineered T-cells (Alvarez-Vallina & Hawkins 1996). However, none have utilised the full length CD28 molecule. There are specific advantages to using full length receptors, such as CD28 as opposed to truncated forms. Full length CD28 is dimeric enabling the receptor to function in its native form, indeed chimeric antigen receptors fail to function optimally when expressed as a monomer (Bridgeman et al. 2010). The invention also provides a targeted costimulatory receptor according to claim 1 which induces signal 2 upon engagement with a cancer-associated or tumour-associated antigen. The full length CD28 molecule contains motifs critical to its native function in binding members of the B7 family of receptors; although this is potentially dangerous from the perspective of CARs carrying CD28 and CD3ζ receptors in tandem, wherein ligation of CAR by B7 could trigger T-cell activation, there are beneficial qualities for receptors harbouring signal 2 receptors alone.

The present inventors have shown that T-lymphocytes (T-cells) can be engineered to express a CoStAR (Costimulatory antigen receptor) to augment T-cell activation upon engagement with a defined tumour associated antigen. The inventors show that activation of T-cells, as measured by IFNγ and IL-2 secretion, by a signal 1 mitogen (OKT3) is enhanced when engineered cells are also permitted to receive signal 2 through the CoStAR upon engagement with the tumour associated antigen CEA. Thus in a first aspect, the present invention provides a recombinant costimulatory antigen receptor (CoStAR) according to the appended claims.
The invention also provides a cellaccording to the appended claims.

The CoStAR is designed such that binding of the CoStAR to its designated tumour antigen results in receptor activation and provides a costimulatory signal to augment concurrent signal 1 mediated signals provided though a recombinant TCR, chimeric antigen receptor (CAR) or endogenous TCR. The tumour antigen may be a tumour associated antigen such as those from the cancer-testis or onco-foetal antigen family of molecules such as CEA or 5T4, and targeted using a single chain antibody fragment. Alternatively, the tumour antigen may be one presented as a peptide via MHC and targeted using a pMHC specific single chain antibody fragment or single chain TCR.

In certain arrangements described herein, the signalling domain comprises a full length (entire protein sequence without the signal peptide) costimulatory receptor, including but not limited to CD2, CD9, CD26, CD27, CD28, CD29, CD38, CD40, CD43, CD46, CD49d, CD55, CD73, CD81, CD82, CD99, CD100, CD134 (OX40), CD137 (41BB), CD150 (SLAM), CD270 (HVEM), CD278 (ICOS), CD357 (GITR), or EphB6. In the present invention, the CoStAR comprises a first intracellular segment comprising an intracellular signaling domain of CD28, and a second intracellular segment comprising an intracellular signaling domain of CD40.

The costimulatory domain may consist of one receptor domain alone, or multiple in tandem (i.e. a fusion receptor) whereby each domain is linked directly or via a flexible linker of up to 50 amino acids in length. In certain embodiments, the signalling domain comprises a fragment of a full length costimulatory receptor wherein the fragment is effective for dimerization. There may be a linker which links the single chain antibody fragment to the signalling domain. This linker if present may be 1-50 amino acids in length. The lymphocyte may be a T cell, including a Tumour Infiltrating Lymphocyte (TIL) a T Regulatory Cell (Treg) or a primary T cell, or an NK cell. In addition to the CoStAR the lymphocyte, T or NK cell, may include a recombinant T-cell receptor (TCR) or Chimeric Antigen Receptor (CAR).

An individual with sufficient experience would know that identifying the exact point at which the signal peptide is cleaved from the mature protein is difficult to determine with accuracy, and although prediction software can be applied, the artificial splicing of sequences upstream of the mature protein may create novel sites for signal peptide peptidases, thus the term 'full length' in this document implies the entire mature protein with up to 5 amino acid deletions or mutations at the very N-terminus of the mature protein, a process critical for optimal splicing of the costimulatory domain into a chimeric receptor. This is in clear distinction from previous sequences published such as that used in Lanitis et al (2013) where only the intracellular domain of CD28 was used, with a CD8 transmembrane domain utilised, and in Krause et al. (1998) where a truncated CD28 receptor was used, truncated to the motif IEV.

There is also described herein a nucleic acid sequence encoding the CoStAR as described above and herein.

There is also described herein a vector which comprises a nucleic acid sequence according to the nucleic acid sequence encoding the CoStAR and, if present, a TCR and/or CAR nucleic acid sequence.

There is also described herein a method for making a lymphocyte, or T or NK cell, according to the disclosure herein, which comprises the step of introducing a nucleic acid encoding the CoStAR or vector, into the lymphocyte.

There is also described herein a pharmaceutical composition which comprises a vector according to that disclosed herein, or lymphocyte (including a T or NK cell) according tothat disclosed herein, together with a pharmaceutically acceptable carrier, diluent or excipient as well as therapeutic uses thereof.

There is also described herein a cell, e.g. a lymphocyte, including a T or NK cell, according to that disclosed herein, or vector according to that disclosed herein, including for use in adoptive cell therapy.

There is also described herein a lymphocyte, including a T or NK cell, according to that disclosed herein, or vector according to that disclosed herein, including for use in a method of treating cancer.

There is also described herein the use of a lymphocyte according to that disclosed herein, or the use of the vector according to that disclosed herein in the manufacture of a medicament for treating cancer.

There is also described herein a lymphocyte according to that disclosed herein, a vector according to that disclosed herein or a pharmaceutical composition as disclosed herein for use in the treatment for cancer.

In some arrangements described herein, the CoStAR receptor comprises a full length CD28 receptor as defined herein. Insome arrangements (but not all claimed), the CoStAR comprises a tumour associated antigen binding domain fused to: a fusion signalling domain comprising or consisting of full length human CD28, such as that shown in SEQ ID NO:2 or a variant thereof having at least 80% sequence identity at the protein level; and one Other moiety selected from: the intracellular domain from human CD137, such as that shown in SEQ ID NO:4 or a variant thereof having at least 80% or 90% sequence identity at the protein level; the intracellular domain from human CD 134, such as that shown in SEQ ID NO:5 or a variant thereof having at least 80% or 90% sequence identity at the protein level; the intracellular domain from human CD2, such as that shown in SEQ ID NO:6 or a variant thereof having at least 80% or 90% sequence identity at the protein level; the intracellular domain from human CD29, such as that shown in SEQ ID NO:7 or a variant thereof having at least 80% or 90% sequence identity at the protein level; the intracellular domain from human GITR, such as that shown in SEQ ID NO:8 or a variant thereof having at least 80% or 90% sequence identity at the protein level; the intracellular domain from human IL2Ry, such as that shown in SEQ ID NO:9 or a variant thereof having at least 80% or 90% sequence identity at the protein level, the intracellular domain from human CD40, such as that shown in SEQ ID NO: 10 or a variant thereof having at least 80% or 90% sequence identity at the protein level; the intracellular domain from human CD150, such as that shown in SEQ ID NO: 11 or a variant thereof having at least 80% or 90% sequence identity at the protein level or the intracellular domain from human CD2 and human CD40, such as that shown in SEQ ID NO: 12 or a variant thereof having at least 80% or 90% sequence identity at the protein level. In an embodiment of the present invention, a CoStAR comprises: (i) a signalling domain comprising or consisting of a full length human CD28 of SEQ ID NO:2 or a variant thereof having at least 80% sequence identity at the protein level wherein said full length human CD28 does not comprise the signal peptide and optionally lacks up to 5 amino acids at the N-terminal of the mature receptor protein once its signal peptide has been cleaved; and (ii) the intracellular domain from human CD40 as shown in SEQ ID NO: 10 or a variant thereof having at least 80% or 90% sequence identity at the protein level, wherein the intracellular domain from human CD40 or a variant thereof has functional activity as a CD40 intracellular signalling domain. In some arrangements, the variant of one of
the moieties as described above lacks 1, 2, 3, 4, or 5 or up to 10 N terminal amino acid residues with reference to a fusion sequence listed above.

### BRIEF DESCRIPTION OF THE DRAWINGS

The following detailed description, given by way of example, but not intended to limit the invention solely to the specific embodiments described, may best be understood in conjunction with the accompanying drawings.

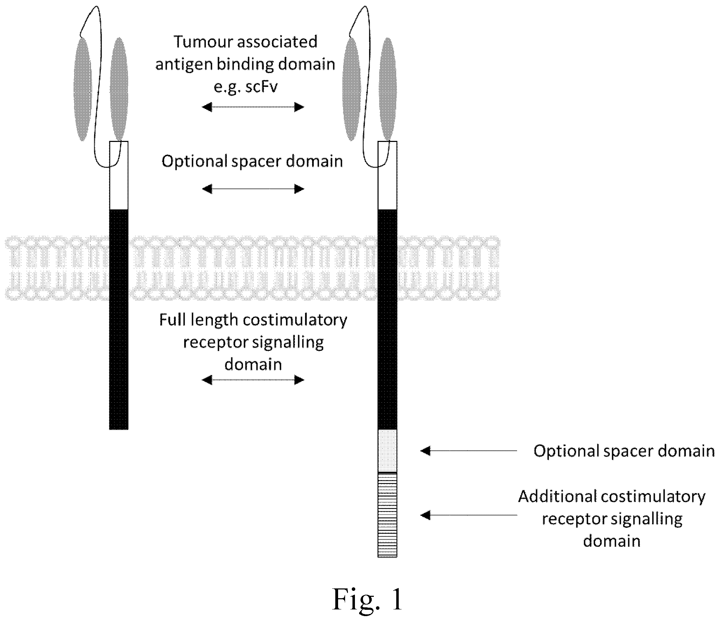

Figure 1 - Structural organisation of single costimulatory and fusion costimulatory domain receptors. A schematic representation of CoStAR receptors set out in the claims is shown. First a CoStAR based on a single costimulatory receptor, and secondly a fusion CoStAR consisting of a full length costimulatory receptor signalling domain fused to a second costimulatory domain.

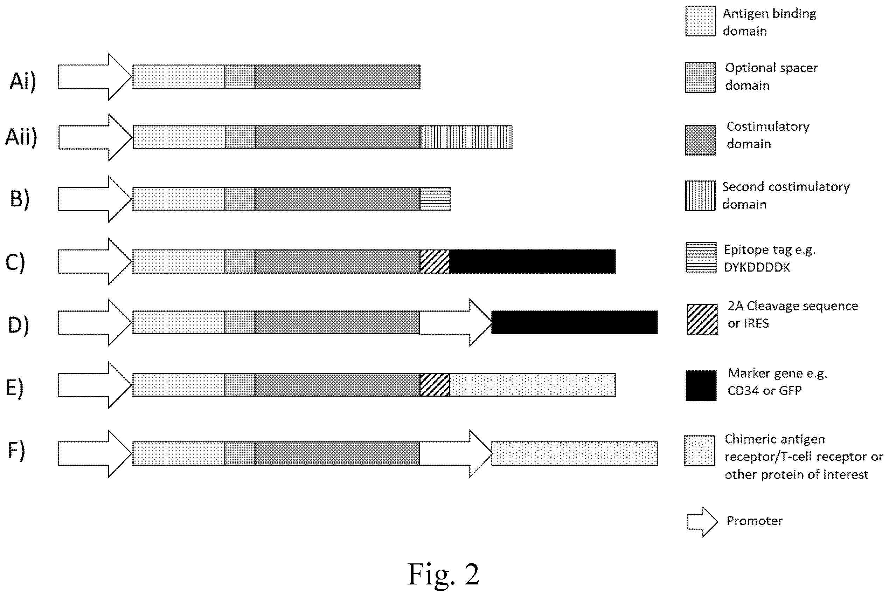

Figure 2 - Genomic organisation of potential CoStAR configurations - The CoStAR consists of an antigen binding domain, an optional spacer domain and a costimulatory domain as shown in figure and described in claims. The CoStAR may be expressed: A) alone from a promoter with the CoStAR consisting of a single (Ai) or fusion (Aii) costimulatory receptor; B) may be expressed with an epitope tag (e.g. His tag, DYKDDDDK (SEQ ID NO: 14) etc) at the N or C-terminus to enable direct staining of the CoStAR; C) along with a marker gene separated using a 2A cleavage sequence or internal ribosomal entry site (IRES); D) along with a marker gene which is expressed from a second promoter; E) along with a protein of interest such as a chimeric antigen receptor or T-cell receptor separated using a 2A cleavage sequence or internal ribosomal entry site (IRES); F) along with a protein of interest such as a chimeric antigen receptor or T-cell receptor which is expressed from a second promoter. It would be clear to an individual with sufficient knowledge that the CoStAR and marker gene/chimeric antigen receptor/T-cell receptor/other protein of interest could be expressed in either orientation or 3' (3-prime) or 5' (5-prime) to one another.

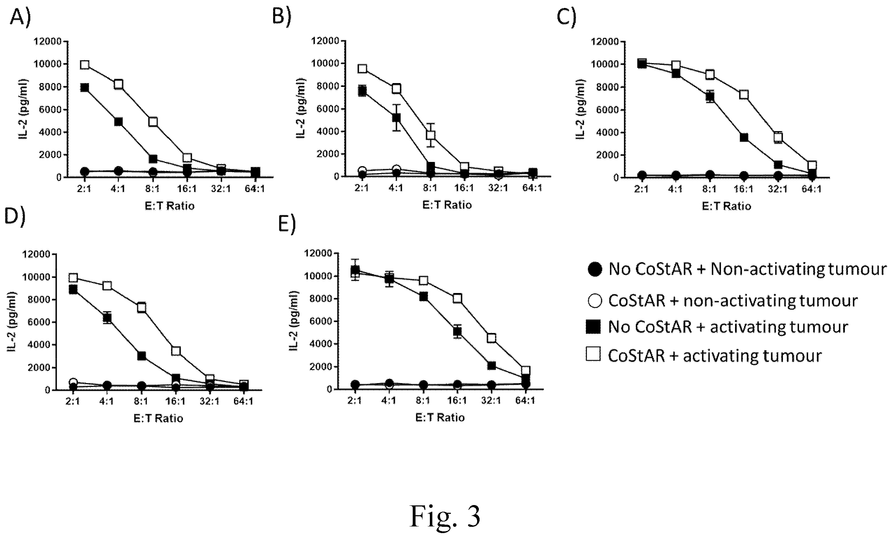

Figure 3 - Functional activity of CoStAR in T-cells in response to LS174T and LoVo tumour presented antigen. Normal donor T-cell populations from donor 1 (A & D), donor 2 (B) and donor 3 (C & E) were lentivirally engineered to express a CoStAR which targets carcinoembryonic antigen and magnetically sorted to enrich for the transgene using CD34 magnetic selection. T-cells were mixed with wild-type un-engineered CEA+ tumour cells (Non-activating tumour) or CEA+ tumour cells engineered to express a cell surface anchored anti-CD3 single chain antibody fragment (Activating tumour) at the indicated effector to target ratios and IL-2 measured in the supernatant by ELISA. Data obtained using LS174T cells (A, B & C) and LoVo (D & E).

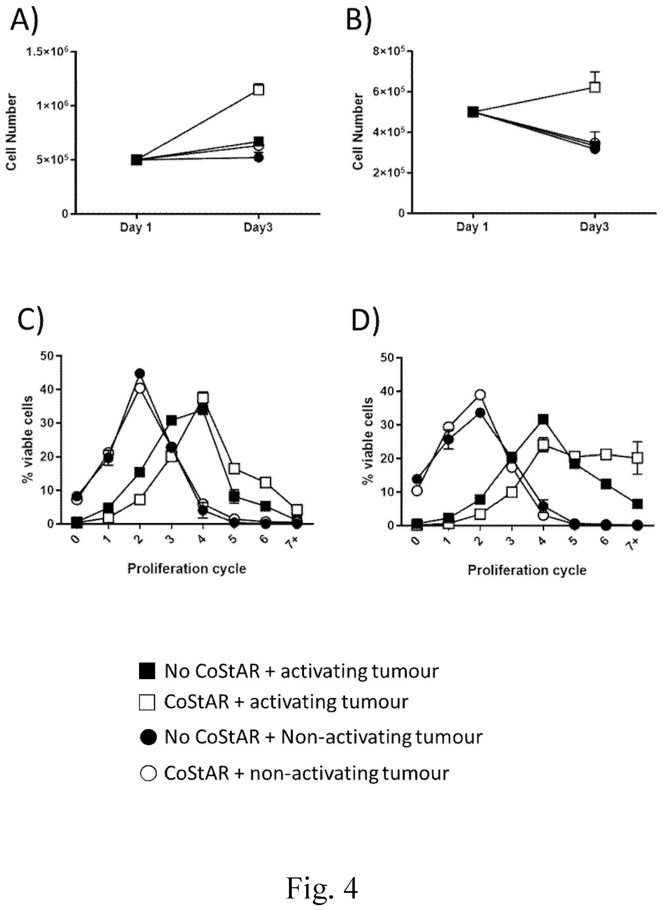

Figure 4 - Effect of CoStAR on T-cell proliferation. 5×10⁵ transduced and non transduced T-cells were mixed with 6.25×10³ wild-type LoVo or LoVo-OKT3 cells in the presence (A) or absence (B) of IL-2 and cell counts made after three days. In another assay under the same cell ratios T-cells from two donors were loaded with proliferation dye and the number of proliferation cycles the cells had gone through determined by dye dilution after six days using flow cytometry.

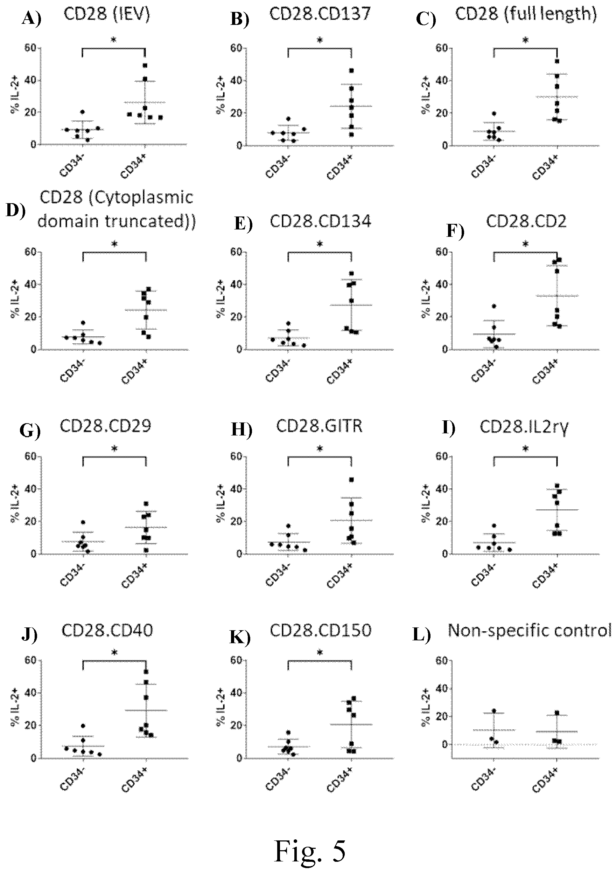

Figure 5 - IL-2 activity of CoStAR fusion receptors in primary human T-cells. Normal donor CD8+ T-cells from seven donors (except control CoStAR is three donors) were lentivirally transduced with the indicated CEA-targeting CoStARs and IL-2 production assessed after an overnight stimulation in the presence of LoVo-OKT3 cells. The proportion of IL-2 positive cells was determined using intracellular flow staining in both the CD34 negative (CoStAR non-transduced) and CD34+ (CoStAR transduced) populations. Asterisks show significant differences between the transduced and non-transduced populations using paired Wilcoxon signed rank test with * p<0.05

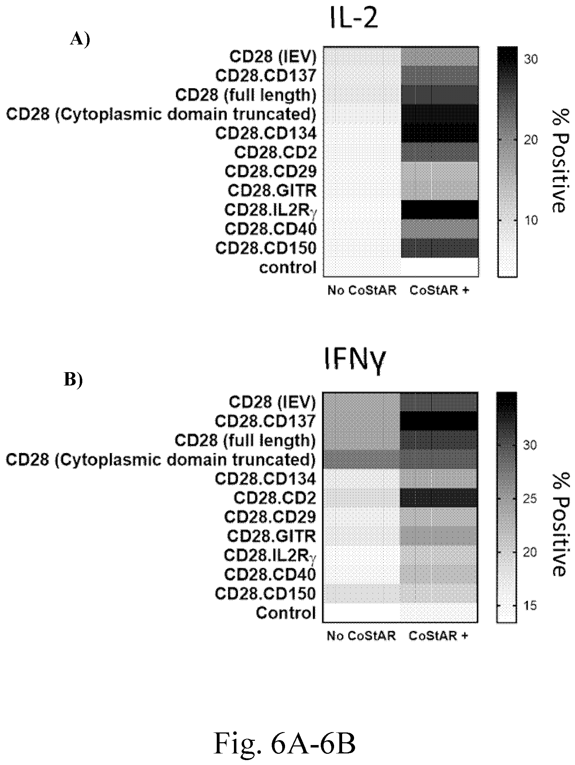

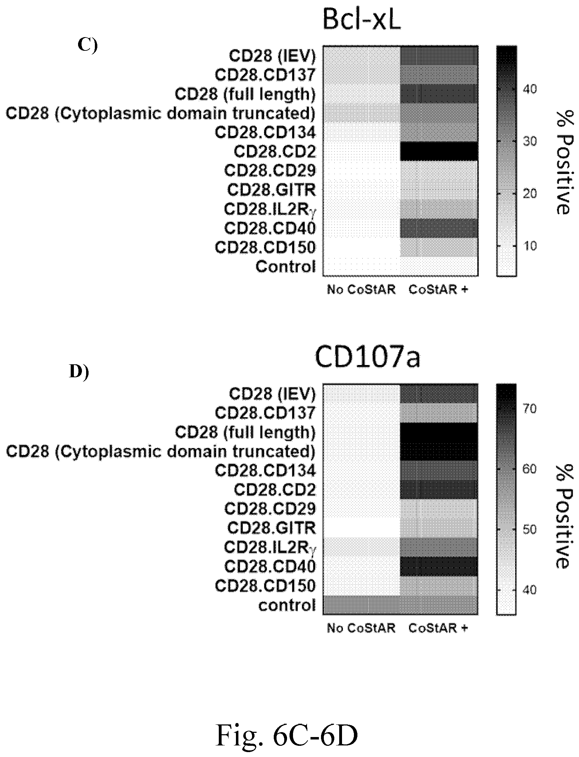

Figure 6A-6D - Multi parameter analysis of CoStAR activity in primary human T-cells. Normal donor CD8+ T-cells were lentivirally transduced with the indicated CEA-targeting CoStARs and IL-2 production assessed after an overnight stimulation in the presence of LoVo-OKT3 cells. The proportion of IL-2 (seven donors) (Fig. 6A), IFNγ (seven donors) (Fig. 6B), bcl-xL (five donors) (Fig. 6C) and CD107a (six donors) (Fig. 6D) positive cells was determined using intracellular flow staining in both the CD34 negative (CoStAR non-transduced) and CD34+ (CoStAR transduced) populations. Control is a non-specific CA125 targeting CoStAR and is from three donors in all instances. Heat maps are averages of all donors with the intensity of colour related to the percentage of cells positive for a particular read out under the defined conditions.

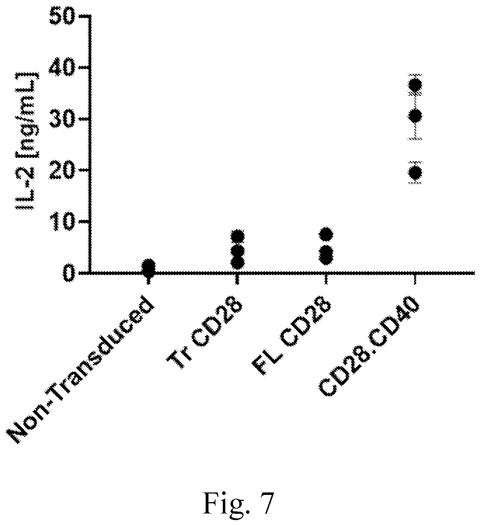

Figure 7 - CD40 enhances IL-2 production from CD28-based CoStARs. Primary human T-cells from three healthy donors were left non-transduced or transduced with either extracellular domain truncated CD28 (Tr CD28), full length CD28 (FL CD28), or CD28.CD40-based CoStARs harbouring a CEA specific scFv (MFE23). Transduced cells were selected using a CD34 marker gene and expanded prior to analysis. T-cells were mixed at an 8:1 effector to target ratio with OKT3 expressing CEA+ LoVo cells for 20 hours before analysis of IL-2 production by ELISA.

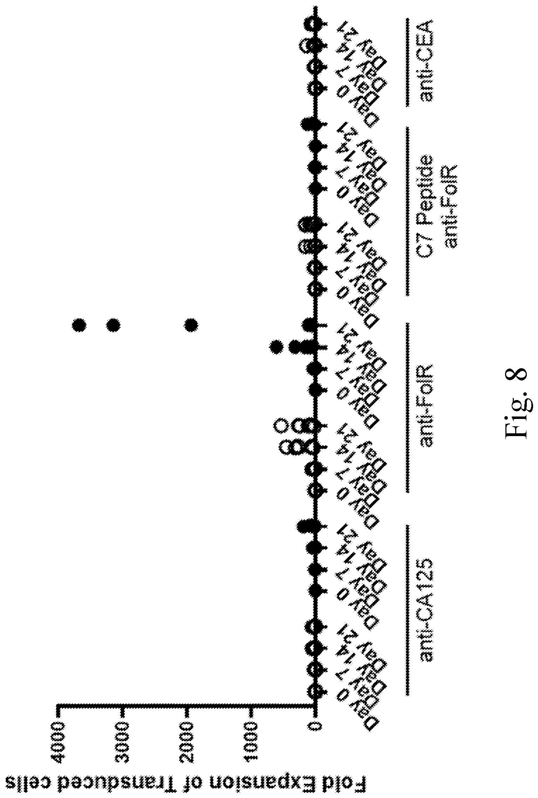

Figure 8 - Effect of signalling domain and target antigen on CoStAR-mediated T-cell expansion. T-cells were transduced with either DYKDDDDK (SEQ ID NO:14) epitope-tagged CD28 or CD28.CD40 based CoStARs harbouring CA125, FolR or CEA specific scFv, or FolR specific binding peptide (C7). T-cells were mixed with OKT3 expressing, CA125+/FolR+/CEA-cell line OVCAR3. The number of transduced cells were counted every 7 days up to 21 days, with fresh OVCAR3 cells added following each count.

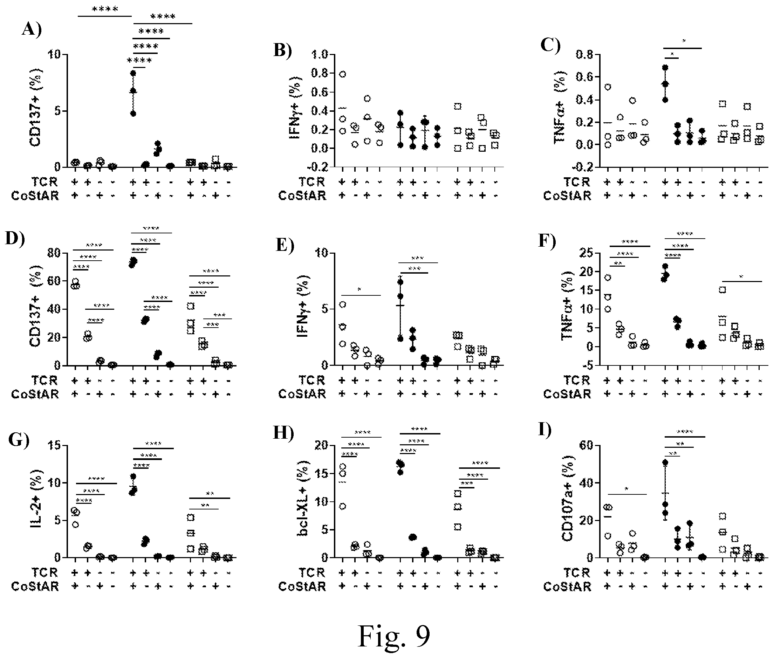

Figure 9 - (A to I) Effect of signalling domain and target antigen on CoStAR-mediated T-cell expansion. T-cells were transduced with either DYKDDDDK (SEQ ID NO: 14) epitope-tagged CD28 or CD28.CD40 based CoStARs harbouring CA125, FolR or CEA specific scFv, or FolR specific binding peptide (C7). T-cells were mixed with OKT3 expressing, CA125+/FolR+/CEA- cell line OVCAR3. The number of transduced cells were counted every 7 days up to 21 days, with fresh OVCAR3 cells added following each count.

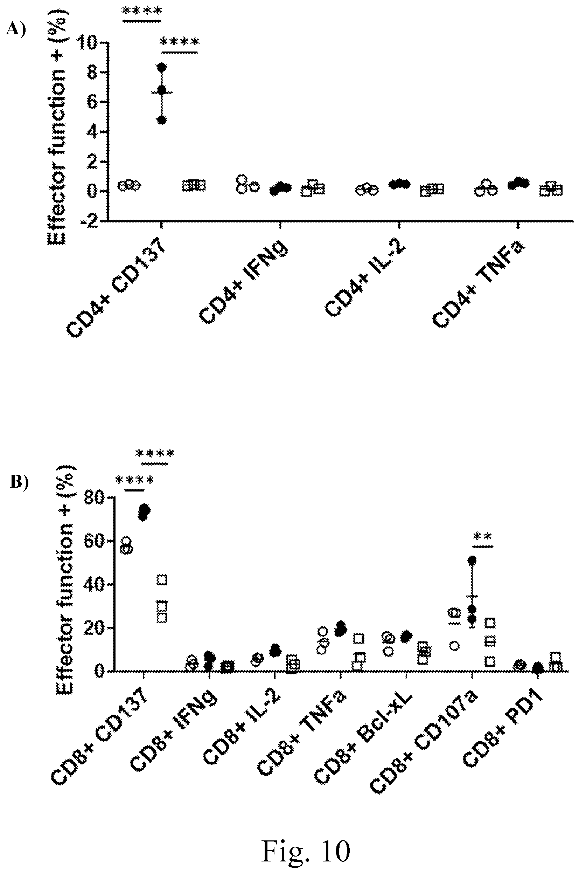

Figure 10 - (A and B) CD40 based CoStARs enhance costimulation of T-cells in a model of TCR-transfer. Primary human T-cells from three healthy donors were transduced with a CEA specific TCR plus either a DYKDDDK-tagged CD28 or CD28.CD40 based CoStAR harbouring either an MFE (open or closed circles) or CA125 (open squares) specific scFv. T-cells were mixed at a 1:1 effector:target ratio with CEA+/CA125- H508 cells and intracellular cytokine staining performed to determine the number of responding CD4+ or CD8+ T-cells in the TCR+/CoStAR+, TCR+/CoStAR-, TCR-/CoStAR+ and TCR-/CoStAR- populations. A 2-way ANOVA (Tukeys test) was performed to determine significant differences in activity: *p>0.05, ** p>0.01, *** p>0.001, **** p>0.0001.

### DETAILED DESCRIPTION OF THE INVENTION

Unless defined otherwise, all technical and scientific terms used herein have the same meaning as is commonly understood by one of skill in the art to which this invention belongs.

### COSTIMULATORY ANTIGEN RECEPTOR (CoStAR)

Provided herein are recombinant costimulatory antigen receptors (CoStARs) comprising: (i) a disease- (in the claims, cancer-) or tumour-associated antigen binding domain; and (ii) an extracellular ligand-binding segment of a stimulatory receptor protein, and (iii) a first intracellular segment comprising an intracellular signaling domain of a receptor protein, and (iv) optionally (but in the claimed invention, not optional) a second intracellular segment comprising an intracellular signaling domain of a second receptor protein. In the present invention, the CoStAR is as defined in claim 1. In certain arrangements (and as claimed), the extracellular segment and the first intracellular segment comprise segments of the same receptor protein. In certain arrangements (not claimed), the extracellular segment and the first intracellular segment comprise segments of different receptor proteins. Incertain arrangements, there is an intervening transmembrane domain between an extracellular segment and the first intracellular segment. Arrangements comprising an optional second intracellular segment and/or an optional third intracellular segment further may comprise spacers between the intracellular segments. In a simple form a CoStAR as described herein comprises a single chain antibody fragment (scFv) fused to full length CD28 that transmits a costimulatory signal dependent upon engagement of the scFv to its cognate antigen and/or engagement of the CD28 to its cognate ligand. As used herein, "full length protein" or "full length receptor" refers to a receptor protein, such as, for example, a CD28 receptor protein. The term "full length" encompasses receptor proteins lacking up to about 5 amino acids or up to 10 amino acids, for example 1, 2, 3, 4, 5, 6, 7, 8, 9, or 10 amino acids, at the N-terminal of the mature receptor protein once its signal peptide has been cleaved. For instance, while a specific cleavage site of a receptors N-terminal signal peptide may be defined, variability in exact point of cleavage has been observed. The term "full length" does not imply presence or absence of amino acids of the receptors N-terminal signal peptide. In one arrngement, the term "full length" (e g. a full length CD28 according to certain disclosures herein) encompasses mature receptor proteins (e.g. CD28 according to certain disclosures herein) lacking the N terminal signal peptide lacking up to about 5, for example 1, 2, 3, 4. 5, or up to 10 amino acids at the N-terminal of the mature receptor protein once its signal peptide has been cleaved. SEQ ID NO. 2 shows the mature CD28 protein including the signal peptide. As mentioned above, a "full length" CD28 receptor according to the various arrangements described herein does not include the signal peptide and may lack up to about 5, for example 1, 2, 3, 4, 5, or up to 10 amino acids at the N-terminal of the mature receptor protein (e.g. N terminal residues N, K, I, L and/or V). This is shown in the exemplary fusions, e g. SEQ ID Nos. 4-12 (note that these may lack up to about 5, for example 1, 2, 3, 4, 5, or up to 10 amino acids at the N-terminal of the mature receptor protein as shown in the boxed region).

CoStARs have modular form and can be constructed to comprise extracellular, transmembrane and intracelluler domains obtained from a one or more proteins, along with the scFv obtained from an antibody that binds to a disease-associated antigen, for example, a tumour associated antigen antigen.

As described herein, a CoStAR comprises a disease-associated, for example a tumour-associated, antigen receptor, such as but not limited to a tumour-associated antigen specific scFv, and a primary costimulatory receptor protein that is capable of binding to its cognate ligand and providing an intracellular signal. In certain arrangements, the primary costimulatory receptor can be less than a full length protein but is sufficient to bind cognate ligand and transduce a signal. In certain arrangements, the primary costimulatory receptor domain is full length, such as but not limited to, full length CD28. Thus, both the antigen specific binding domain and the ligand specific receptor are capable of binding cognate antigen and ligand respectively. Figure 1 depicts two arrangements of CoStAR constructs. Both CoStARs comprise an antigen binding domain, an optional spacer, and a full-length costimulatory receptor protein comprising an extracellular ligand binding segment and intracellular signaling domain. In another arrangement, a CoStAR comprises an antigen binding domain, an optional spacer, an extracellular ligand-binding portion of a costimulatory receptor protein, a transmembrane domain, and an intracellular signaling domain of a second, different costimulatory receptor protein. In certain arrangements, the extracellular ligand-binding portion comprises a CD28 truncation, for example, a C-terminal CD28 truncation after amino acids IEV as in SEQ ID NO: 13 and is followed by an intracellular signalling domain. In certain arrangements, the intracellular signalling domain is from CD40. The transmembrane separating the extracellular ligand-binding and intracellular signalling domains can be from, with limitation, CD28, CD40. By comparison to the left construct of Fig. 1, the right construct of Fig. 1 further comprises an additional second intracellular receptor signalling domain and an optional spacer. In further arrangements, CoStARs can comprise additional costimulatory domains, for example a third, intracellular costimulatory signaling domain and in this respect may be similar to crtain chimeric antigen receptors (CARs), which have been classified into first (CD3ζ only), second (one costimulatory domain + CD3ζ), or third generation (more than one costimulatory domain + CD3ζ).

Costimulatory receptor proteins useful in CoStARs of the present disclosure include, without limitation, CD2, CD9, CD26, CD27, CD28, CD29, CD38, CD40, CD43, CD46, CD49d, CD55, CD73, CD81, CD82, CD99, CD100, CD134 (OX40), CD137 (41BB), CD150 (SLAM), CD270 (HVEM), CD278 (ICOS), CD357 (GITR), or EphB6, which in their natural form comprise extracellular ligand binding domains and intracellular signal transducing domains. In the present invention, CD28 and CD40 are ustilised. For example, CD2 is characterized as a cell adhesion molecule found on the surface of T cells and is capable of initiating intracellular signals necessary for T cell activation. CD27 is characterized as a type II transmembrane glycoprotein belonging to the TNF superfamily (TNFSF) whose expression on B cells is induced by antigen-receptor activation in B cells. CD28 is one of the proteins on T cells and is the receptor for CD80 (B7.1) and CD86 (B7.2) ligands on antigen-presenting cells. CD137 (4-1BB) ligand is found on most leukocytes and on some non-immune cells. OX4 ligand is expressed on many antigen-presenting cells such as DC2s (dendritic cells), macrophages, and B lymphocytes. In one arrangement, the costimulatory receptor protein is full length CD28 as defined herein.

In arrangements described herein, a CoStAR may be expressed alone under the control of a promoter in a therapeutic population of cells that have therapeutic activity, for example, Tumour Infiltrating Lymphocytes (TILs). Alternatively, the CoStAR may be expressed along with a therapeutic transgene such as a chimeric antigen receptor (CAR) and/or T-cell Receptor (TCR). , for example as described in SEQ ID NOS:3-12 (note that may lack up to about 5, for example 1, 2, 3, 4, 5, or up to 10 amino acids at the N-terminal of the mature receptor protein). Thus, the present disclosure also relates to CoStar constructs having a sequence as shown in any of SEQ ID NOs:3-12, including one of these sequences which lacks up to about 5, for example 1, 2, 3, 4, 5, or up to 10 amino acids at the N-terminal of the mature receptor protein).. Suitable TCRs and CARs are well known in the literature, for example HLA-A*02-NYESO-1 specific TCRs (Rapoport et al. Nat Med 2015) or anti-CD19scFv.CD3ζ fusion CARs (Kochenderfer et al. J Clin Oncol 2015) which have been successfully used to treat Myeloma or B-cell malignancies respectively. The CoStARs described herein may be expressed with any known CAR or TCR thus providing the cell with a regulatable growth switch to allow cell expansion in-vitro or in-vivo, and a conventional activation mechanism in the form of the TCR or CAR for anti-cancer activity. Thus the present disclosure provides a cell for use in adoptive cell therapy comprising a CoStAR as described herein and a TCR and/or CAR that specifically binds to a tumour associated antigen. An exemplary CoStAR comprising CD28 includes an extracellular antigen binding domain and an extracellular, transmembrane and intracellular signaling domain which comprises the amino acid sequence shown as SEQ ID NO:2.

The term "antigen binding domain" as used herein refers to an antibody fragment including, but not limited to, a diabody, a Fab, a Fab', a F(ab')2, an Fv fragment, a disulfide stabilized Fv fragment (dsFv), a (dsFv)2, a bispecific dsFv (dsFv-dsFv'), a disulfide stabilized diabody (ds diabody), a single-chain antibody molecule (scFv), an scFv dimer (bivalent diabody), a multispecific antibody formed from a portion of an antibody comprising one or more CDRs, a camelized single domain antibody, a nanobody, a domain antibody, a bivalent domain antibody, or any other antibody fragment that binds to an antigen but does not comprise a complete antibody structure. An antigen binding domain is capable of binding to the same antigen to which the parent antibody or a parent antibody fragment (e.g., a parent scFv) binds. In some embodiments, an antigen-binding fragment may comprise one or more complementarity detrmining regions (CDRs) from a particular human antibody grafted to frameworks (FRs) from one or more different human antibodies.

The antigen binding domain can be made specific for any disease-associated antigen, including but not limited to tumour-associated antigens (TAAs) and infectious disease-associated antigens. In certain embodiments, the ligand binding domain is bispecific. Antigens have been identified in most of the human cancers, including Burkitt lymphoma, neuroblastoma, melanoma, osteosarcoma, renal cell carcinoma, breast cancer, prostate cancer, lung carcinoma, and colon cancer. TAA's include, without limitation, CD19, CD20, CD22, CD24, CD33, CD38, CD123, CD228, CD138, BCMA, GPC3, CEA, folate receptor (FRα), mesothelin, CD276, gp100, 5T4, GD2, EGFR, MUC-1, PSMA, EpCAM, MCSP, SM5-1, MICA, MICB, ULBP and HER-2. TAAs further include neoantigens, peptide/MHC complexes, and HSP/peptide complexes.

In certain embodiments, the antigen binding domain comprises a T-cell receptor or binding fragment thereof that binds to a defined tumour specific peptide-MHC complex. The term "T cell receptor," or "TCR," refers to a heterodimeric receptor composed of αβ or γδ chains that pair on the surface of a T cell. Each α, β, γ, and δ chain is composed of two Ig-like domains: a variable domain (V) that confers antigen recognition through the complementarity determining regions (CDR), followed by a constant domain (C) that is anchored to cell membrane by a connecting peptide and a transmembrane (TM) region. The TM region associates with the invariant subunits of the CD3 signalling apparatus. Each of the V domains has three CDRs. These CDRs interact with a complex between an antigenic peptide bound to a protein encoded by the major histocompatibility complex (pMHC) (Davis and Bjorkman (1988) Nature, 334, 395-402; Davis et al. (1998) Annu Rev Immunol, 16, 523-544; Murphy (2012), xix, 868 p.).

In certain embodiments, the antigen binding domain comprises a natural ligand of a tumour expressed protein or tumour-binding fragment thereof. For example, the transferrin receptor 1 (TfR1), also known as CD71, is a homodimeric protein that is a key regulator of cellular iron homeostasis and proliferation. Although TfR1 is expressed at a low level in a broad variety of cells, it is expressed at higher levels in rapidly proliferating cells, including malignant cells in which overexpression has been associated with poor prognosis. In an embodiment of the invention, the antigen binding domain comprises transferrin or a transferrin receptor-binding fragment thereof.

In certain embodiments, the antigen binding domain is specific to a defined tumour associated antigen, such as but not limited to FRα, CEA, 5T4, CA125, SM5-1 or CD71. In certain embodiments, the tumour associated antigen can be a tumour-specific peptide-MHC complex. In certain such embodiments, the peptide is a neoantigen. In other embodiments, the tumour associated antigen it a peptide-heat shock protein complex.

In an arrangements as discussed herein, the CoStAR extracellular, transmembrane and intracellular domain signalling domain has the sequence shown as SEQ ID NO: 1.

As use herein, the term "specifically binds" or "is specific for" refers to measurable and reproducible interactions, such as binding between a target and an antibody or antibody moiety, that is determinative of the presence of the target in the presence of a heterogeneous population of molecules, including biological molecules. For example, an antibody moiety that specifically binds to a target (which can be an epitope) is an antibody moiety that binds the target with greater affinity, avidity, more readily, and/or with greater duration than its bindings to other targets. In some arrangements, an antibody moiety that specifically binds to an antigen reacts with one or more antigenic determinants of the antigen (for example a cell surface antigen or a peptide/MHC protein complex) with a binding affinity that is at least about 10 times its binding affinity for other targets.

### SPACER DOMAIN

A CoStAR as described herein optionally comprises a spacer region between the antigen binding domain and the costimulatory receptor. As used herein, the term "spacer" refers to the extracellular structural region of a CoStAR that separates the antigen binding domain from the external ligand binding domain of the costimulatory protein. The spacer provides flexibility to access the targeted antigen and receptor ligand. In certain arrangements long spacers are employed, for example to target membrane-proximal epitopes or glycosylated antigens (see Guest R.D. et al. The role of extracellular spacer regions in the optimal design of chimeric immune receptors: evaluation of four different scFvs and antigens. J. Immunother. 2005;28:203-211; Wilkie S. et al., Retargeting of human T cells to tumor-associated MUC1: the evolution of a chimeric antigen receptor. J. Immunol. 2008;180:4901-4909). In other arrangements, CoStARs bear short spacers, for example to target membrane distal epitopes (see Hudecek M. et al., Receptor affinity and extracellular domain modifications affect tumor recognition by ROR1-specific chimeric antigen receptor T cells. Clin. Cancer Res. 2013;19:3153-3164; Hudecek M. et al., The nonsignaling extracellular spacer domain of chimeric antigen receptors is decisive for in vivo antitumor activity. Cancer Immunol. Res. 2015;3:125-135). In certain arrangement, the spacer comprises all or part of or is derived from an IgG hinge, including but not limited to IgG1, IgG2, or IgG4. By "derived from an Ig hinge" is meant a spacer comprising insertions, deletions, or mutations in an IgG hinge. In certain arrangements, a spacer can comprise all or part of one or more antibody constant domains, such as but not limited to CH2 and/or CH3 domains. In certain arrangements, in a spacer comprising all or part of a CH2 domain, the CH2 domain is modified so as not to bind to an Fc receptor. For example, Fc receptor binding in myeloid cells has been found to impair CAR T cell functionality. In certain arrangements, the spacer comprises all or part of an Ig-like hinge from CD28, CD8, or other protein comprising a hinge region. In certain arrangements that comprise a spacer, the spacer is from 1 and 50 amino acids in length.

### SIGNALLING DOMAIN

As discussed above, in certain arrangements (not all claimed), a CoStAR comprises a full length primary costimulatory receptor which can comprise an extracellular ligand binding and intracellular signalling portion of, without limitation, CD2, CD9, CD26, CD27, CD28, CD29, CD38, CD40, CD43, CD46, CD49d, CD55, CD73, CD81, CD82, CD99, CD100, CD134 (OX40), CD137 (41BB), CD150 (SLAM), CD270 (HVEM), CD278 (ICOS), CD357 (GITR), or EphB6. In the present invention, the CoStAR comprises an extracellular ligand binding and intracellular signalling portion of CD28. In other arrangements, the costimulatory receptor comprises a chimeric protein, for instance comprising an extracellular ligand binding domain of one of the aforementioned proteins and an intracellular signalling domain of another of the aforementioned proteins. In the present invention, the CoStAR comprises an extracellular ligand-binding segment of a CD28, and a first intracellular segment comprising an intracellular signaling domain of CD28. In certain arrangements (not claimed), the signalling portion of the CoStAR comprises a single signalling domain. In other arrangements, the signalling portion of the CoStAR comprises a second intracellular signalling domain such as but not limited to: CD2, CD27, CD28, CD40, CD134 (OX40), CD137 (4-1BB), CD150 (SLAM). In the present invention, the CoStAR comprises a second intracellular signalling domain from CD40. In certain arrangements (not claimed), the first and second intracellular signalling domains are the same. In other arrangements, the the first and second intracellular signalling domains are different. In the present invention, the CoStAR comprises a first intracellular signalling domain from CD28 and a second intracellular signalling domain from CD40. In certain arrangements, the costimulatory receptor is capable of dimerization. Without being bound by theory, it is thought that CoStARs dimerize or associate with other accessory molecules for signal initiation. In certain arrangements, CoStARs dimerize or associate with accessory molecules through transmembrane domain interactions. In certain arrangements, dimerization or association with accessory molecules is assisted by costimulatory receptor interactions in the intracellular portion, and/or the extracellular portion of the costimulatory receptor.

### TRANSMEMBRANE DOMAIN

Although the main function of the transmembrane is to anchor the CoStAR in the T cell membrane, in certain arrangements, the transmembrane domain influences CoStAR function. In certain arrangements, the transmembrane domain is comprised by the full length primary costimulatory receptor domain. In arrangements disclosed herein wherein the CoStAR construct comprises an extracellular domain of one receptor and an intracellular signalling domain of a second receptor, the transmembrane domain can be that of the extracellular domain or the intracellular domain. In certain arrangements, the transmembrane domain is from CD4, CD8α, CD28, or ICOS. Gueden et al. associated use of the ICOS transmembrane domain with increased CAR T cell persistence and overall anti-tumor efficacy (Guedan S. et al., Enhancing CAR T cell persistence through ICOS and 4-1BB costimulation. JCI Insight. 2018;3:96976). In an arrangement, the transmembrane domain comprises a hydrophobic α helix that spans the cell membrane.

Examples of CoStAR signaling domains are provided as SEQ ID NOs. 3-12.

### Variants

In some arrangements, amino acid sequence variants of the antibody moieties provided herein are contemplated. For example, it may be desirable to improve the binding affinity and/or other biological properties of the antibody moiety. Amino acid sequence variants of an antibody moiety may be prepared by introducing appropriate modifications into the nucleotide sequence encoding the antibody moiety, or by peptide synthesis. Such modifications include, for example, deletions from, and/or insertions into and/or substitutions of residues within the amino acid sequences of the antibody moiety. Any combination of deletion, insertion, and substitution can be made to arrive at the final construct, provided that the final construct possesses the desired characteristics, e.g., antigen-binding.
In some arrangements, antibody binding domain moieties comprising one or more amino acid substitutions, deletions, or insertions are provided. Sites of interest for mutational changes include the antibody binding domain heavy and light chain variable regions (VRs) and frameworks (FRs). Amino acid substitutions may be introduced into a binding domain of interest and the products screened for a desired activity, e.g., retained/improved antigen binding or decreased immunogenicity. In certain arrangements, amino acid substitutions may be introduced into one or more of the primary co-stimulatory receptor domain (extracellular or intracellular), secondary costimulatory receptor domain, or extracellular co-receptor domain. Accordingly, the disclosed herein are CoStAR proteins and component parts particularly disclosed herein as well as variants thereof, i.e. CoStAR proteins and component parts having at least 75%, at least 80%, at least 85%, at least 87%, at least 90%, at least 91%, at least 92%, at least 93%, at least 94%, at least 95%, at least 96%, at least 97%, at least 98%, at least 99% sequence identity to the amino acid sequences particulary disclosed herein. The terms "percent similarity," "percent identity," and "percent homology" when referring to a particular sequence are used as set forth in the University of Wisconsin GCG software program BestFit. Other algorithms may be used, e.g. BLAST, psiBLAST or TBLASTN (which use the method of Altschul et al. (1990) J. Mol. Biol. 215: 405-410), FASTA (which uses the method of Pearson and Lipman (1988) PNAS USA 85: 2444-2448),

Particular amino acid sequence variants may differ from a reference sequence by insertion, addition, substitution or deletion of 1 amino acid, 2, 3, 4, 5-10, 10-20 or 20-30 amino acids. In some arrangements, a variant sequence may comprise the reference sequence with 1, 2, 3, 4, 5, 6, 7, 8, 9, 10 or more residues inserted, deleted or substituted. For example, 5, 10,15, up to 20, up to 30 or up to 40 residues may be inserted, deleted or substituted.

In some preferred arrangements, a variant may differ from a reference sequence by 1, 2, 3, 4, 5, 6, 7, 8, 9, 10 or more conservative substitutions. Conservative substitutions involve the replacement of an amino acid with a different amino acid having similar properties. For example, an aliphatic residue may be replaced by another aliphatic residue, a non-polar residue may be replaced by another non-polar residue, an acidic residue may be replaced by another acidic residue, a basic residue may be replaced by another basic residue, a polar residue may be replaced by another polar residue or an aromatic residue may be replaced by another aromatic residue. Conservative substitutions may, for example, be between amino acids within the following groups:

Conservative substitutions are shown in the Table below.

| Original Residue | Exemplary Substitutions | Preferred Substitutions |
|---|---|---|
| Ala (A) | Val; Leu; Ile | Val |
| Arg (R) | Lys; Gln; Asn | Lys |
| Asn (N) | Gln; His; Asp; Lys; Arg | Gln |
| Asp (D) | Glu; Asn | glu |
| Cys (C) | Ser; Ala | ser |
| Gln (Q) | Asn; Glu | Asn |
| Glu (E) | Asp; Gln | Asp |
| Gly (G) | Ala | Ala |
| His (H) | Asn; Gin; Lys; Arg | Arg |
| Ile (I) | Leu; Val; Met; Ala; Phe; Norleucine | Leu |
| Leu (L) | Norleucinne; Ile; Val; Met; Ala; Phe | Ile |

| | | |
|---|---|---|
| Lys (K) | Arg; Gln; Asn | Arg |
| Met (M) | Leu; Phe; Ile | Leu |
| Phe (F) | Trp; Leu; Val; Ile; Ala; Tyr | Tyr |
| Pro (P) | Ala | Ala |
| Ser (S) | Thr | Thr |
| Thr (T) | Val; Ser | Ser |
| Trp (W) | Tyr; Phe | Tyr |
| Tyr (Y) | Trp; Phe; Thr; Ser | Phe |
| Val (V) | Ile; Leu; Met; Phe; Ala; Norleucine | Leu |

Amino acids may be grouped into different classes according to common side-chain properties: a. hydrophobic: Norleucine, Met, Ala, Val, Leu, Ile; b. neutral hydrophilic: Cys, Ser, Thr, Asn, Gln; c. acidic: Asp, Glu; d. basic: His, Lys, Arg; e. residues that influence chain orientation: Gly, Pro; aomatic: Trp, Tyr, Phe. Non-conservative substitutions will entail exchanging a member of one of these classes for another class.

### CELLS

The cells used in the present invention may be any lymphocyte that is useful in adoptive cell therapy, such as a T-cell or a natural killer (NK) cell, an NKT cell, a gamma/delta T-cell or T regulatory cell. The cells may be allogeneic or autologous to the patient.

T cells or T lymphocytes are a type of lymphocyte that have a central role in cell-mediated immunity. They can be distinguished from other lymphocytes, such as B cells and natural killer cells (NK cells), by the presence of a T-cell receptor (TCR) on the cell surface. There are various types of T cell, as summarised below. Cytotoxic T cells (TC cells, or CTLs) destroy virally infected cells and tumour cells, and are also implicated in transplant rejection. CTLs express the CD8 molecule at their surface.

These cells recognize their targets by binding to antigen associated with MHC class I, which is present on the surface of all nucleated cells. Through IL-10, adenosine and other molecules secreted by regulatory T cells, the CD8+ cells can be inactivated to an anergic state, which prevent autoimmune diseases such as experimental autoimmune encephalomyelitis.

Memory T cells are a subset of antigen-specific T cells that persist long-term after an infection has resolved. They quickly expand to large numbers of effector T cells upon re-exposure to their cognate antigen, thus providing the immune system with "memory" against past infections. Memory T cells comprise three subtypes: central memory T cells (TCM cells) and two types of effector memory T cells (TEM cells and TEMRA cells). Memory cells may be either CD4+ or CD8+. Memory T cells typically express the cell surface protein CD45RO. Regulatory T cells (Treg cells), formerly known as suppressor T cells, are crucial for the maintenance of immunological tolerance. Their major role is to shut down T cell-mediated immunity toward the end of an immune reaction and to suppress auto-reactive T cells that escaped the process of negative selection in the thymus.

Two major classes of CD4+ Treg cells have been described - naturally occurring Treg cells and adaptive Treg cells. Naturally occurring Treg cells (also known as CD4⁺CD25⁺FoxP3⁺ Treg cells) arise in the thymus and have been linked to interactions between developing T cells with both myeloid (CD11c⁺) and plasmacytoid (CD123⁺) dendritic cells that have been activated with TSLP. Naturally occurring Treg cells can be distinguished from other T cells by the presence of an intracellular molecule called FoxP3. Adaptive Treg cells (also known as Tr1 cells or Th3 cells) may originate during a normal immune response.

Natural Killer Cells (or NK cells) are a type of cytolytic cell which form part of the innate immune system. NK cells provide rapid responses to innate signals from virally infected cells in an MHC independent manner. NK cells (belonging to the group of innate lymphoid cells) are defined as large granular lymphocytes (LGL) and constitute the third kind of cells differentiated from the common lymphoid progenitor generating B and T lymphocytes.

In certain arrangements disclosed herein, therapeutic cells comprise autologous cells engineered to express a CoStAR. In certain arrangements, therapeutic cells comprise allogeneic cells engineered to express a CoStAR. Autologous cells expressing CoStARs may be advantageous in avoiding graft-versus-host disease (GVDH) due to TCR-mediated recognition of recipient alloantigens. Also, the immune system of a CoStAR recipient could attack the infused CoStAR cells, causing rejection. In certain arrangements, to prevent GVHD, and to reduce rejection, endogenous TcR is removed from allogeneic CoStAR cells by genome editing.

### NUCLEIC ACIDS

An aspect of the present disclosure provides a nucleic acid sequence, encoding any of the CoStARs, polypeptides, or proteins described herein (including functional portions and functional variants thereof). As used herein, the terms "polynucleotide", "nucleotide", and "nucleic acid" are intended to be synonymous with each other. It will be understood by a skilled person that numerous different polynucleotides and nucleic acids can encode the same polypeptide as a result of the degeneracy of the genetic code. In addition, it is to be understood that skilled persons may, using routine techniques, make nucleotide substitutions that do not affect the polypeptide sequence encoded by the polynucleotides described here to reflect the codon usage of any particular host organism in which the polypeptides are to be expressed, e.g. codon optimisation. Nucleic acids according to the invention may comprise DNA or RNA. They may be single stranded or double-stranded. They may also be polynucleotides which include within them synthetic or modified nucleotides. A number of different types of modification to oligonucleotides are known in the art. These include methylphosphonate and phosphorothioate backbones, addition of acridine or polylysine chains at the 3' and/or 5' ends of the molecule. For the purposes of the present disclosure, it is to be understood that the polynucleotides may be modified by any method available in the art. Such modifications may be carried out in order to enhance the in vivo activity or life span of polynucleotides of interest.

The terms "variant", "homologue" or "derivative" in relation to a nucleotide sequence include any substitution of, variation of, modification of, replacement of, deletion of or addition of one (or more) nucleic acid from or to the sequence.
The nucleic acid sequence may encode the protein sequence shown as SEQ ID NO:2 or a variant thereof. The nucleotide sequence may comprise the codon optimised human CD28 nucleic acid sequence shown in SEQ ID NO: 1 or variants thereof (represented in Fig. 1).

The present disclosure also provides a nucleic acid sequence which comprises a nucleic acid sequence encoding a CoStAR and a further nucleic acid sequence encoding a T-cell receptor (TCR) and/or chimeric antigen receptor (CAR).

The nucleic acid sequences may be joined by a sequence allowing co-expression of the two or more nucleic acid sequences. For example, the construct may comprise an internal promoter, an internal ribosome entry sequence (IRES) sequence or a sequence encoding a cleavage site. The cleavage site may be self-cleaving, such that when the polypeptide is produced, it is immediately cleaved into the discrete proteins without the need for any external cleavage activity. Various self-cleaving sites are known, including the Foot-and Mouth disease virus (FMDV) and the 2A self-cleaving peptide. The co-expressing sequence may be an internal ribosome entry sequence (IRES). The co-expressing sequence may be an internal promoter.

### VECTORS

The present disclosure provides a vector which comprises a nucleic acid sequence or nucleic acid construct of the present disclosure.

Such a vector may be used to introduce the nucleic acid sequence(s) or nucleic acid construct(s) into a host cell so that it expresses one or more CoStAR(s) according to the first aspect of the invention and, optionally, one or more other proteins of interest (POI), for example a TCR or a CAR. The vector may, for example, be a plasmid or a viral vector, such as a retroviral vector or a lentiviral vector, or a transposon-based vector or synthetic mRNA.

The nucleic acids of the present disclosure may also be used for nucleic acid immunization and gene therapy, using standard gene delivery protocols. Methods for gene delivery are known in the art. See, e.g., U.S. Pat. Nos. 5,399,346, 5,580,859, 5,589,466.

Vectors derived from retroviruses, such as the lentivirus, are suitable tools to achieve long-term gene transfer since they allow long-term, stable integration of a transgene or transgenes and its propagation in daughter cells. The vector may be capable of transfecting or transducing a lymphocyte including a T cell or an NK cell. The present invention also provides vectors in which a nucleic acid of the present invention is inserted. The expression of natural or synthetic nucleic acids encoding a CoStAR, and optionally a TCR or CAR is typically achieved by operably linking a nucleic acid encoding the CoStAR and TCR/CAR polypeptide or portions thereof to one or more promoters, and incorporating the construct into an expression vector.

Additional promoter elements, e.g., enhancers, regulate the frequency of transcriptional initiation. Typically, these are located in the region 30-110 bp upstream of the start site, although a number of promoters have recently been shown to contain functional elements downstream of the start site as well. The spacing between promoter elements frequently is flexible, so that promoter function is preserved when elements are inverted or moved relative to one another. In the thymidine kinase (tk) promoter, the spacing between promoter elements can be increased to 50 bp apart before activity begins to decline.

One example of a suitable promoter is the immediate early cytomegalovirus (CMV) promoter sequence. This promoter sequence is a strong constitutive promoter sequence capable of driving high levels of expression of any polynucleotide sequence operatively linked thereto. Another example of a suitable promoter is Elongation Growth Factor-1α (EF-1α). However, other constitutive promoter sequences may also be used, including, but not limited to the simian virus 40 (SV40) early promoter, mouse mammary tumour virus (MMTV), human immunodeficiency virus (HIV) long terminal repeat (LTR) promoter, MoMuLV promoter, an avian leukemia virus promoter, an Epstein-Barr virus immediate early promoter, a Rous sarcoma virus promoter, as well as human gene promoters such as, but not limited to, the actin promoter, the myosin promoter, the hemoglobin promoter, and the creatine kinase promoter.

The vectors can be suitable for replication and integration in eukaryotic cells. Typical cloning vectors contain transcription and translation terminators, initiation sequences, and promoters useful for regulation of the expression of the desired nucleic acid sequence. Viral vector technology is well known in the art and is described, for example, in Sambrook et al. (2001, Molecular Cloning: A Laboratory Manual, Cold Spring Harbor Laboratory, New York), and in other virology and molecular biology manuals, see also, WO 01/96584; WO 01/29058; and U.S. Pat. No.6,326,193). In some arrangements, the constructs are as shown in SEQ ID NOS:3-12 (diagramatically represented in Fig. 2). In some arrangements the nucleic acids are multi-cistronic constructs that permit the expression of multiple transgenes (e.g., CoStAR and a TCR and/or CAR etc.) under the control of a single promoter. In some arrangements, the transgenes (e.g., CoStAR and a TCR and/or CAR etc.) are separated by a self-cleaving 2A peptide. Examples of 2A peptides useful in the nucleic acid constructs disclosed herein include F2A, P2A, T2A and E2A. In other arrangements, the nucleic acid construct is a multi-cistronic construct comprising two promoters; one promoter driving the expression of CoStAR and the other promoter driving the expression of the TCR or CAR. In some arrangements, the dual promoter constructs are uni-directional. In other arrangements, the dual promoter constructs are bi-directional. In order to assess the expression of the CoStAR polypeptide or portions thereof, the expression vector to be introduced into a cell can also contain either a selectable marker gene or a reporter gene or both to facilitate identification and selection of expressing cells from the population of cells sought to be transfected or transduced through viral vectors.

### SOURCES OF CELLS

Prior to expansion and genetic modification, a source of cells (e.g., immune effector cells, e.g., T cells or NK cells) is obtained from a subject. The term "subject" is intended to include living organisms in which an immune response can be elicited (e.g., mammals). Examples of subjects include humans. Also included are dogs, cats, mice, rats and transgenic species thereof. T cells can be obtained from a number of sources, including peripheral blood mononuclear cells, bone marrow, lymph node tissue, cord blood, thymus tissue, tissue from a site of infection, ascites, pleural effusion, spleen tissue, and tumours.

In one arrangment, T cells are isolated from peripheral blood lymphocytes by lysing the red blood cells and depleting the monocytes, for example, by centrifugation through a PERCOLLTM gradient or by counterflow centrifugal elutriation.

A specific subpopulation of T cells, such as CD3+, CD28+, CD4+, CD8+, CD45RA+, and CD45RO+T cells, can be further isolated by positive or negative selection techniques. For example, in one arrangement, T cells are isolated by incubation with anti-CD3/anti-CD28-conjugated beads, such as DYNABEADS^{®} M-450 CD3/CD28 T, for a time period sufficient for positive selection of the desired T cells. In one arrangement, the time period is about 30 minutes. In a further arrangement, the time period ranges from 30 minutes to 36 hours or longer and all integer values there between. In a further arrangement, the time period is at least 1, 2, 3, 4, 5, or 6 hours. In yet another preferred arrangement, the time period is 10 to 24 hours. In one arrangement, the incubation time period is 24 hours. Longer incubation times may be used to isolate T cells in any situation where there are few T cells as compared to other cell types, such in isolating tumour infiltrating lymphocytes (TIL) from tumour tissue or from immunocompromised individuals. Further, use of longer incubation times can increase the efficiency of capture of CD8+ T cells. Thus, by simply shortening or lengthening the time T cells are allowed to bind to the CD3/CD28 beads and/or by increasing or decreasing the ratio of beads to T cells (as described further herein), subpopulations of T cells can be preferentially selected for or against at culture initiation or at other time points during the process. Additionally, by increasing or decreasing the ratio of anti-CD3 and/or anti-CD28 antibodies on the beads or other surface, subpopulations of T cells can be preferentially selected for or against at culture initiation or at other desired time points. The skilled artisan would recognize that multiple rounds of selection can also be used in the context of this disclosure. In certain arrangements, it may be desirable to perform the selection procedure and use the "unselected" cells in the activation and expansion process. "Unselected" cells can also be subjected to further rounds of selection.

Enrichment of a T cell population by negative selection can be accomplished with a combination of antibodies directed to surface markers unique to the negatively selected cells. One method is cell sorting and/or selection via negative magnetic immunoadherence or flow cytometry that uses a cocktail of monoclonal antibodies directed to cell surface markers present on the cells negatively selected. For example, to enrich for CD4+ cells by negative selection, a monoclonal antibody cocktail typically includes antibodies to CD14, CD20, CD16, HLA-DR, and CD8. In certain arrangements, it may be desirable to enrich for or positively select for regulatory T cells which typically express CD4+, CD25+, CD62Lhi, GITR+, CD137, PD1, TIM3, LAG-3, CD150 and FoxP3+. Alternatively, in certain arrangements, T regulatory cells are depleted by anti-CD25 conjugated beads or other similar method of selection.

The methods described herein can include, e.g., selection of a specific subpopulation of immune effector cells, e.g., T cells, that are a T regulatory cell-depleted population, CD25+ depleted cells, using, e.g., a negative selection technique, e.g., described herein. Preferably, the population of T regulatory depleted cells contains less than 30%, 25%, 20%, 15%, 10%, 5%, 4%, 3%, 2%, 1% of CD25+ cells.

A specific subpopulation of CoStAR effector cells that specifically bind to a target antigen can be enriched for by positive selection techniques. For example, in some arrangements, effector cells are enriched for by incubation with target antigen-conjugated beads for a time period sufficient for positive selection of the desired abTCR effector cells. In some arrangements, the time period is about 30 minutes. In some arrangements, the time period ranges from 30 minutes to 36 hours or longer (including all ranges between these values). In some arrangements, the time period is at least one, 2, 3, 4, 5, or 6 hours. In some arrangements, the time period is 10 to 24 hours. In some arrangements, the incubation time period is 24 hours. For isolation of effector cells present at low levels in the heterogeneous cell population, use of longer incubation times, such as 24 hours, can increase cell yield. Longer incubation times may be used to isolate effector cells in any situation where there are few effector cells as compared to other cell types. The skilled artisan would recognize that multiple rounds of selection can also be used in the context of this disclosure.

T cells for stimulation can also be frozen after a washing step. After the washing step that removes plasma and platelets, the cells may be suspended in a freezing solution. While many freezing solutions and parameters are known in the art and will be useful in this context, one method involves using PBS containing 20% DMSO and 8% human serum albumin, or culture media containing 10% Dextran 40 and 5% Dextrose, 20% Human Serum Albumin and 7.5% DMSO, or 31.25% Plasmalyte-A, 31.25% Dextrose 5%, 0.45% NaCl, 10% Dextran 40 and 5% Dextrose, 20% Human Serum Albumin, and 7.5% DMSO or other suitable cell freezing media containing for example, Hespan and PlasmaLyte A, the cells then are frozen to -80°C at a rate of 1° per minute and stored in the vapor phase of a liquid nitrogen storage tank. Other methods of controlled freezing may be used as well as uncontrolled freezing immediately at -20° C or in liquid nitrogen.

### Allogeneic CoStAR

In arrangements described herein, the immune effector cell can be an allogeneic immune effector cell, e.g., T cell or NK cell. For example, the cell can be an allogeneic T cell, e.g., an allogeneic T cell lacking expression of endogenous T cell receptor (TCR) and/or human leukocyte antigen (HLA), e.g., HLA class I and/or HLA class II.

A T cell lacking a functional endogenous TCR can be, e.g., engineered such that it does not express any functional TCR on its surface, engineered such that it does not express one or more subunits that comprise a functional TCR (e.g., engineered such that it does not express (or exhibits reduced expression) of TCR alpha, TCR beta, TCR gamma, TCR delta, TCR epsilon, and/or TCR zeta) or engineered such that it produces very little functional TCR on its surface. Alternatively, the T cell can express a substantially impaired TCR, e.g., by expression of mutated or truncated forms of one or more of the subunits of the TCR. The term "substantially impaired TCR" means that this TCR will not elicit an adverse immune reaction in a host.

A T cell described herein can be, e.g., engineered such that it does not express a functional HLA on its surface. For example, a T cell described herein, can be engineered such that cell surface expression HLA, e.g., HLA class 1 and/or HLA class II, is downregulated. In some arrangements, downregulation of HLA may be accomplished by reducing or eliminating expression of beta- 2 microglobulin (B2M).

In some arrangements disclosed herein, the T cell can lack a functional TCR and a functional HLA, e.g., HLA class I and/or HLA class II. Modified T cells that lack expression of a functional TCR and/or HLA can be obtained by any suitable means, including a knock out or knock down of one or more subunit of TCR or HLA. For example, the T cell can include a knock down of TCR and/or HLA using siRNA, shRNA, clustered regularly interspaced short palindromic repeats (CRISPR) transcription-activator like effector nuclease (TALEN), or zinc finger endonuclease (ZFN).

In some arrangements, the allogeneic cell can be a cell which does not expresses or expresses at low levels an inhibitory molecule, e.g. a cell engineered by any method described herein. For example, the cell can be a cell that does not express or expresses at low levels an inhibitory molecule, e.g., that can decrease the ability of a CoStAR-expressing cell to mount an immune effector response. Examples of inhibitory molecules include PD1, PD-L1, PD-L2, CTLA4, TIM3, CEACAM (e.g., CEACAM-1, CEACAM-3 and/or CEACAM-5), LAG3, VISTA, BTLA, TIGIT, LAIR1, CD160, 2B4, CD80, CD86, B7-H3 (CD276), B7-H4 (VTCN1), HVEM (TNFRSF14 or CD270), KIR, A2aR, MHC class I, MHC class II, Gal9, adenosine, and TGFR beta. Inhibition of an inhibitory molecule, e.g., by inhibition at the DNA, RNA or protein level, can optimize a CAR-expressing cell performance. In arrangements, an inhibitory nucleic acid, e.g., an inhibitory nucleic acid, e.g., a dsRNA, e.g., an siRNA or shRNA, a clustered regularly interspaced short palindromic repeats (CRISPR), a transcription-activator like effector nuclease (TALEN), or a zinc finger endonuclease (ZFN), e.g., as described herein, can be used.

### siRNA and shRNA to inhibit endogenous TCR or HLA

In some arrangements, TCR expression and/or HLA expression can be inhibited using siRNA or shRNA that targets a nucleic acid encoding a TCR and/or HLA, and/or an inhibitory molecule described herein (e.g., PD1, PD-L1, PD-L2, CTLA4, TIM3, CEACAM (e.g., CEACAM-1, CEACAM-3 and/or CEACAM-5), LAG3, VISTA, BTLA, TIGIT, LAIR1, CD160, 2B4, CD80, CD86, B7-H3 (CD276), B7-H4 (VTCN1), HVEM (TNFRSF14 or CD270), KIR, A2aR, MHC class I, MHC class II, Gal9, adenosine, and TGFR beta), in a T cell.

Expression of siRNA and shRNAs in T cells can be achieved using any conventional expression system, e.g., such as a lentiviral expression system. Exemplary shRNAs that downregulate expression of components of the TCR are described, e.g., in US Publication No.: 2012/0321667. Exemplary siRNA and shRNA that downregulate expression of HLA class I and/or HLA class II genes are described, e.g., in U.S. publication No.: US 2007/0036773.

### CRISPR to inhibit TCR or HLA

"CRISPR" or "CRISPR to inhibit TCR and/or HLA" as used herein refers to a set of clustered regularly interspaced short palindromic repeats, or a system comprising such a set of repeats. "Cas", as used herein, refers to a CRISPR-associated protein. A "CRISPR/Cas" system refers to a system derived from CRISPR and Cas which can be used to silence or mutate a TCR and/or HLA gene, and/or an inhibitory molecule described herein (e.g., PD1, PD-L1, PD-L2, CTLA4, TIM3, CEACAM (e.g., CEACAM-1, CEACAM-3 and/or CEACAM-5), LAG3, VISTA, BTLA, TIGIT, LAIR1, CD160, 2B4, CD80, CD86, B7-H3 (CD276), B7-H4 (VTCNl), HVEM (TNFRSF14 or CD270), KIR, A2aR, MHC class I, MHC class II, GAL9, adenosine, and TGFR beta).

Naturally-occurring CRISPR/Cas systems are found in approximately 40% of sequenced eubacteria genomes and 90% of sequenced archaea. Grissa et al. (2007) BMC Bioinformatics 8: 172. This system is a type of prokaryotic immune system that confers resistance to foreign genetic elements such as plasmids and phages and provides a form of acquired immunity. Barrangou et al. (2007) Science 315: 1709-1712; Marragini et al. (2008) Science 322: 1843-1845.

### Activation and Expansion of T Cells

T cells may be activated and expanded generally using methods as described, for example, in U.S. Patents 6,352,694; 6,534,055; 6,905,680; 6,692,964; 5,858,358; 6,887,466; 6,905,681; 7,144,575; 7,067,318; 7,172,869; 7,232,566; 7,175,843; 5,883,223; 6,905,874; 6,797,514; 6,867,041; and U.S. Patent Application Publication No. 20060121005.

Generally, the T cells of the invention may be expanded by contact with a surface having attached thereto an agent that stimulates a CD3/TCR complex associated signal and a ligand that stimulates a costimulatory molecule on the surface of the T cells. In particular, T cell populations may be stimulated as described herein, such as by contact with an anti-CD3 antibody, or antigen-binding fragment thereof, or an anti-CD2 antibody immobilized on a surface, or by contact with a protein kinase C activator (e.g., bryostatin) in conjunction with a calcium ionophore. For co-stimulation of an accessory molecule on the surface of the T cells, a ligand that binds the accessory molecule is used. For example, a population of T cells can be contacted with an anti-CD3 antibody and an anti-CD28 antibody, under conditions appropriate for stimulating proliferation of the T cells. To stimulate proliferation of either CD4+ T cells or CD8+ T cells, an anti-CD3 antibody and an anti-CD28 antibody can be used. Examples of an anti-CD28 antibody include 9.3, B-T3, XR-CD28 (Diaclone, Besancon, France) can be used as can other methods commonly known in the art (Berg et al., Transplant Proc. 30(8):3975-3977, 1998; Haanen et al., J. Exp. Med. 190(9): 13191328,1999; Garland et al., J. Immunol Meth. 227(l-2):53-63, 1999).

In some arrangements, expansion can be performed using flasks or containers, or gas-permeable containers known by those of skill in the art and can proceed for 7 days, 8 days, 9 days, 10 days, 11 days, 12 days, 13 days, or 14 days, about 7 days to about 14 days, about 8 days to about 14 days, about 9 days to about 14 days, about 10 days to about 14 days, about 11 days to about 14 days, about 12 days to about 14 days, or about 13 days to about 14 days. In some arrangements, the second TIL expansion can proceed for about 14 days.

In certain arrangements, the expansion can be performed using non-specific T-cell receptor stimulation in the presence of interleukin-2 (IL-2) or interleukin-15 (IL-15). The non-specific T-cell receptor stimulus can include, for example, an anti-CD3 antibody, such as about 30 ng/ml of OKT3, a mouse monoclonal anti-CD3 antibody (commercially available from Ortho-McNeil, Raritan, N.J. or Miltenyi Biotech, Auburn, Calif.) or UHCT-1 (commercially available from BioLegend, San Diego, Calif., USA). CoStAR cells can be expanded in vitro by including one or more antigens, including antigenic portions thereof, such as epitope(s), of a cancer, which can be optionally expressed from a vector, such as a human leukocyte antigen A2 (HLA-A2) binding peptide, e.g., 0.3 .mu.M MART-1:26-35 (27 L) or gpl 00:209-217 (210M), optionally in the presence of a T-cell growth factor, such as 300 IU/mL IL-2 or IL-15. Other suitable antigens may include, e.g., NY-ESO-1, TRP-1, TRP-2, tyrosinase cancer antigen, MAGE-A3, SSX-2, and VEGFR2, or antigenic portions thereof. CoStAR cells may also be rapidly expanded by re-stimulation with the same antigen(s) of the cancer pulsed onto HLA-A2-expressing antigen-presenting cells. Alternatively, the CoStAR cells can be further stimulated with, e.g., example, irradiated, autologous lymphocytes or with irradiated HLA-A2+ allogeneic lymphocytes and IL-2. In some embodiments, the stimulation occurs as part of the expansion. In some embodiments, the expansion occurs in the presence of irradiated, autologous lymphocytes or with irradiated HLA-A2+ allogeneic lymphocytes and IL-2.

In certain arrangements, the cell culture medium comprises IL-2. In some arrangements, the cell culture medium comprises about 1000 IU/mL, about 1500 IU/mL, about 2000 IU/mL, about 2500 IU/mL, about 3000 IU/mL, about 3500 IU/mL, about 4000 IU/mL, about 4500 IU/mL, about 5000 IU/mL, about 5500 IU/mL, about 6000 IU/mL, about 6500 IU/mL, about 7000 IU/mL, about 7500 IU/mL, or about 8000 IU/mL, or between 1000 and 2000 IU/mL, between 2000 and 3000 IU/mL, between 3000 and 4000 IU/mL, between 4000 and 5000 IU/mL, between 5000 and 6000 IU/mL, between 6000 and 7000 IU/mL, between 7000 and 8000 IU/mL, or between 8000 IU/mL of IL-2.

In certain arrangements, the cell culture medium comprises OKT3 antibody. In some arrangements, the cell culture medium comprises about 0.1 ng/mL, about 0.5 ng/mL, about 1 ng/mL, about 2.5 ng/mL, about 5 ng/mL, about 7.5 ng/mL, about 10 ng/mL, about 15 ng/mL, about 20 ng/mL, about 25 ng/mL, about 30 ng/mL, about 35 ng/mL, about 40 ng/mL, about 50 ng/mL, about 60 ng/mL, about 70 ng/mL, about 80 ng/mL, about 90 ng/mL, about 100 ng/mL, about 200 ng/mL, about 500 ng/mL, about 1 µg/mL or between 0.1 ng/mL and 1 ng/mL, between 1 ng/mL and 5 ng/mL, between 5 ng/mL and 10 ng/mL, between 10 ng/mL and 20 ng/mL, between 20 ng/mL and 30 ng/mL, between 30 ng/mL and 40 ng/mL, between 40 ng/mL and 50 ng/mL, or between 50 ng/mL and 100 ng/mL of OKT3 antibody.

In ceratain arrangements, a combination of IL-2, IL-7, IL-15, and/or IL-21 are employed as a combination during the expansion. In some arrangements, IL-2, IL-7, IL-15, and/or IL-21 as well as any combinations thereof can be included during the expansion. In some arrangements, a combination of IL-2, IL-15, and IL-21 are employed as a combination during the expansion. In some arrangements, IL-2, IL-15, and IL-21 as well as any combinations thereof can be included.

In ceratin arrangements, the expansion can be conducted in a supplemented cell culture medium comprising IL-2, OKT-3, and antigen-presenting feeder cells.

In certain arrangements, the expansion culture media comprises about 500 IU/mL of IL-15, about 400 IU/mL of IL-15, about 300 IU/mL of IL-15, about 200 IU/mL of IL-15, about 180 IU/mL of IL-15, about 160 IU/mL of IL-15, about 140 IU/mL of IL-15, about 120 IU/mL of IL-15, or about 100 IU/mL of IL-15, orabout 500 IU/mL of IL-15 to about 100 IU/mL of IL-15, or about 400 IU/mL of IL-15 to about 100 IU/mL of IL-15 or about 300 IU/mL of IL-15 to about 100 IU/mL of IL-15 or about 200 IU/mL of IL-15, or about 180 IU/mL of IL-15.

In some arrangements, the expansion culture media comprises about 20 IU/mL of IL-21, about 15 IU/mL of IL-21, about 12 IU/mL of IL-21, about 10 IU/mL of IL-21, about 5 IU/mL of IL-21, about 4 IU/mL of IL-21, about 3 IU/mL of IL-21, about 2 IU/mL of IL-21, about 1 lU/mL of IL-21, or about 0.5 IU/mL of IL-21, or about 20 IU/mL of IL-21 to about 0.5 IU/mL of IL-21, or about 15 IU/mL of IL-21 to about 0.5 IU/mL of IL-21, or about 12 IU/mL of IL-21 to about 0.5 IU/mL of IL-21, or about 10 lU/mL of IL-21 to about 0.5 IU/mL of IL-21, or about 5 IU/mL of IL-21 to about 1 IU/mL of IL-21, or about 2 IU/mL of IL-21. In some arrangements, the cell culture medium comprises about 1 IU/mL of IL-21, or about 0.5 IU/mL of IL-21.

In some arrangements the antigen-presenting feeder cells (APCs) are PBMCs. In an arrangement, the ratio of CoStAR cells to PBMCs and/or antigen-presenting cells in the expansion is about 1 to 25, about 1 to 50, about 1 to 100, about 1 to 125, about 1 to 150, about 1 to 175, about 1 to 200, about 1 to 225, about 1 to 250, about 1 to 275, about 1 to 300, about 1 to 325, about 1 to 350, about 1 to 375, about 1 to 400, or about 1 to 500, or between 1 to 50 and 1 to 300, or between 1 to 100 and 1 to 200.

In certain arrangements, the primary stimulatory signal and the costimulatory signal for the T cell may be provided by different protocols. For example, the agents providing each signal may be in solution or coupled to a surface. When coupled to a surface, the agents may be coupled to the same surface (i.e., in "cis" formation) or to separate surfaces (i.e., in "trans" formation). Alternatively, one agent may be coupled to a surface and the other agent in solution. In one arrangement, the agent providing the costimulatory signal is bound to a cell surface and the agent providing the primary activation signal is in solution or coupled to a surface. In certain arrangements, both agents can be in solution. In one arrangement, the agents may be in soluble form, and then cross-linked to a surface, such as a cell expressing Fc receptors or an antibody or other binding agent which will bind to the agents. In this regard, see for example, U.S. Patent Application Publication Nos. 20040101519 and 20060034810 for artificial antigen presenting cells (aAPCs) that are contemplated for use in activating and expanding T cells in the present disclosure.

In one arrangement, the two agents are immobilized on beads, either on the same bead, i.e., "cis," or to separate beads, i.e., "trans." By way of example, the agent providing the primary activation signal is an anti-CD3 antibody or an antigen-binding fragment thereof and the agent providing the costimulatory signal is an anti-CD28 antibody or antigen-binding fragment thereof; and both agents are co -immobilized to the same bead in equivalent molecular amounts. In one arrangement, a 1:1 ratio of each antibody bound to the beads for CD4+ T cell expansion and T cell growth is used. In certain aspects of the present disclosure, a ratio of anti CD3:CD28 antibodies bound to the beads is used such that an increase in T cell expansion is observed as compared to the expansion observed using a ratio of 1:1. In one particular arrangement an increase of from about 1 to about 3 fold is observed as compared to the expansion observed using a ratio of 1:1. In one arrangement, the ratio of CD3:CD28 antibody bound to the beads ranges from 100:1 to 1:100 and all integer values there between. In one arrangement of the present disclosure, more anti-CD28 antibody is bound to the particles than anti-CD3 antibody, i.e., the ratio of CD3:CD28 is less than one. In certain arrangements of the disclosure, the ratio of anti CD28 antibody to anti CD3 antibody bound to the beads is greater than 2:1. In one particular arrangement, a 1:100 CD3:CD28 ratio of antibody bound to beads is used. In one arrangement, a 1:75 CD3:CD28 ratio of antibody bound to beads is used. In a further arrangement, a 1:50 CD3:CD28 ratio of antibody bound to beads is used. In one arrangement, a 1:30 CD3:CD28 ratio of antibody bound to beads is used. In one preferred arrangement, a 1:10 CD3:CD28 ratio of antibody bound to beads is used. In one arrangement, a 1:3 CD3:CD28 ratio of antibody bound to the beads is used. In yet one arrangement, a 3:1 CD3:CD28 ratio of antibody bound to the beads is used.

Ratios of particles to cells from 1:500 to 500:1 and any integer values in between may be used to stimulate T cells or other target cells. As those of ordinary skill in the art can readily appreciate, the ratio of particles to cells may depend on particle size relative to the target cell. For example, small sized beads could only bind a few cells, while larger beads could bind many. In certain arrangements the ratio of cells to particles ranges from 1: 100 to 100:1 and any integer values in-between and in further arrangements the ratio comprises 1:9 to 9:1 and any integer values in between, can also be used to stimulate T cells. The ratio of anti-CD3- and anti-CD28-coupled particles to T cells that result in T cell stimulation can vary as noted above, however certain preferred values include 1:100, 1:50, 1:40, 1:30, 1:20, 1:10, 1:9, 1:8, 1:7, 1:6, 1:5, 1:4, 1:3, 1:2, 1:1, 2:1, 3:1, 4:1, 5:1, 6:1, 7:1, 8:1, 9:1, 10:1, and 15:1 with one preferred ratio being at least 1:1 particles per T cell. In one arrangement, a ratio of particles to cells of 1: 1 or less is used. In one particular arrangement, a preferred particle: cell ratio is 1:5. In further arrangements, the ratio of particles to cells can be varied depending on the day of stimulation. For example, in one arrangement, the ratio of particles to cells is from 1:1 to 10:1 on the first day and additional particles are added to the cells every day or every other day thereafter for up to 10 days, at final ratios of from 1: 1 to 1:10 (based on cell counts on the day of addition). In one particular arrangement, the ratio of particles to cells is 1:1 on the first day of stimulation and adjusted to 1:5 on the third and fifth days of stimulation. In one arrangement, particles are added on a daily or every other day basis to a final ratio of 1:1 on the first day, and 1:5 on the third and fifth days of stimulation. In one arrangement, the ratio of particles to cells is 2:1 on the first day of stimulation and adjusted to 1:10 on the third and fifth days of stimulation. In one arrangement, particles are added on a daily or every other day basis to a final ratio of 1:1 on the first day, and 1:10 on the third and fifth days of stimulation. One of skill in the art will appreciate that a variety of other ratios may be suitable for use in the present disclosure. In particular, ratios will vary depending on particle size and on cell size and type. In one arrangement, the most typical ratios for use are in the neighborhood of 1:1, 2: 1 and 3:1 on the first day.

In further arrangements of the present disclosure, the cells, such as T cells, are combined with agent-coated beads, the beads and the cells are subsequently separated, and then the cells are cultured. In an alternative arrangement, prior to culture, the agent-coated beads and cells are not separated but are cultured together. In a further arrangement, the beads and cells are first concentrated by application of a force, such as a magnetic force, resulting in increased ligation of cell surface markers, thereby inducing cell stimulation.

### PREPARATION OF CoStAR CELLS

Viral- and non-viral-based genetic engineering tools can be used to generate CoStAR T cells, resulting in permanent or transient expression of therapeutic genes. Retrovirus-based gene delivery is a mature, well-characterized technology, which has been used to permanently integrate CARs into the host cell genome (Scholler J., e.g. Decade-long safety and function of retroviral-modified chimeric antigen receptor T cells. Sci. Transl. Med. 2012;4:132ra53; Rosenberg S.A. et al., Gene transfer into humans-immunotherapy of patients with advanced melanoma, using tumor-infiltrating lymphocytes modified by retroviral gene transduction. N. Engl. J. Med. 1990;323:570-578).

Non-viral DNA transfection methods can also be used. For example, Singh et al describes use of the Sleeping Beauty (SB) transposon system developed to engineer CAR T cells (Singh H., et al., Redirecting specificity of T-cell populations for CD19 using the Sleeping Beauty system. Cancer Res. 2008;68:2961-2971) and is being used in clinical trials (see e.g., ClinicalTrials.gov: NCT00968760 and NCT01653717). The same technology is applicable to engineer CoStARs cells.

Multiple SB enzymes have been used to deliver transgenes. Mátés describes a hyperactive transposase (SB100X) with approximately 100-fold enhancement in efficiency when compared to the first-generation transposase. SB100X supported 35-50% stable gene transfer in human CD34(+) cells enriched in hematopoietic stem or progenitor cells. (Mátés L. et al., Molecular evolution of a novel hyperactive Sleeping Beauty transposase enables robust stable gene transfer in vertebrates. Nat. Genet. 2009;41:753-761) and multiple transgenes can be delivered from multicistronic single plasmids (e.g., Thokala R. et al., Redirecting specificity of T cells using the Sleeping Beauty system to express chimeric antigen receptors by mix-and-matching of VL and VH domains targeting CD123+ tumors. PLoS ONE. 2016;11:e0159477) or multiple plasmids (e.g., Hurton L.V. et al., Tethered IL-15 augments antitumor activity and promotes a stem-cell memory subset in tumor-specific T cells. Proc. Natl. Acad. Sci. USA. 2016;113:E7788-E7797). Such systems are used with CoStARs of the invention.

Morita et al, describes the piggyBac transposon system to integrate larger transgenes (Morita D. et al., Enhanced expression of anti-CD19 chimeric antigen receptor in piggyBac transposon-engineered T cells. Mol. Ther. Methods Clin. Dev. 2017;8:131-140) Nakazawa et al. describes use of the system to generate EBV-specific cytotoxic T-cells expressing HER2-specific chimeric antigen receptor (Nakazawa Y et al, PiggyBac-mediated cancer immunotherapy using EBV-specific cytotoxic T-cells expressing HER2-specific chimeric antigen receptor. Mol. Ther. 2011;19:2133-2143). Manuri et al used the system to generate CD-19 specific T cells (Manuri P.V.R. et al., piggyBac transposon/transposase system to generate CD19-specific T cells for the treatment of B-lineage malignancies. Hum. Gene Ther. 2010;21:427-437).

Transposon technology is easy and economical. One potential drawback is the longer expansion protocols currently employed may result in T cell differentiation, impaired activity and poor persistence of the infused cells. Monjezi et al describe development minicircle vectors that minimize these difficulties through higher efficiency integrations (Monjezi R. et al., Enhanced CAR T-cell engineering using non-viral Sleeping Beauty transposition from minicircle vectors. Leukemia. 2017;31:186-194). These transposon technologies can be used for CoStARs of the invention.

### PHARMACEUTICAL COMPOSITIONS

The present disclosure also relates to a pharmaceutical composition containing a vector or a CoStAR expressing cell of the disclosure together with a pharmaceutically acceptable carrier, diluent or excipient, and optionally one or more further pharmaceutically active polypeptides and/or compounds.

In some arrangements, a pharmaceutical composition is provided comprising a CoStAR described above and a pharmaceutically acceptable carrier. In some arrangements, a pharmaceutical composition is provided comprising a nucleic acid encoding a CoStAR according to any of the arrangements described above and a pharmaceutically acceptable carrier. In some arrangements, a pharmaceutical composition is provided comprising an effector cell expressing a CoStAR described above and a pharmaceutically acceptable carrier. Such a formulation may, for example, be in a form suitable for intravenous infusion.

As used herein, by "pharmaceutically acceptable" or "pharmacologically compatible" is meant a material that is not biologically or otherwise undesirable, e.g., the material may be incorporated into a pharmaceutical composition to be administered to a patient without causing any significant undesirable biological effects or interacting in a deleterious manner with any of the other components of the composition in which it is contained. Pharmaceutically acceptable carriers or excipients have preferably met the required standards of toxicological and manufacturing testing and/or are included on the Inactive Ingredient Guide prepared by the U.S. Food and Drug administration.

An aspect of the invention provides a population of modified T cells expressing a recombinant CoStAR. A suitable population may be produced by a method described above.

The population of modified T cells may be for use as a medicament. For example, a population of modified T cells as described herein may be used in cancer immunotherapy therapy, for example adoptive T cell therapy.

Other arrangements provide the use of a population of modified T cells as described herein for the manufacture of a medicament for the treatment of cancer, a population of modified T cells as described herein for the treatment of cancer, and a method of treatment of cancer (not claimed) may comprise administering a population of modified T cells as described herein to an individual in need thereof.

The population of modified T cells may be autologous i.e. the modified T cells were originally obtained from the same individual to whom they are subsequently administered (i.e. the donor and recipient individual are the same). A suitable population of modified T cells for administration to the individual may be produced by a method comprising providing an initial population of T cells obtained from the individual, modifying the T cells to express a cAMP PDE or fragment thereof and an antigen receptor which binds specifically to cancer cells in the individual, and culturing the modified T cells.

The population of modified T cells may be allogeneic i.e. the modified T cells were originally obtained from a different individual to the individual to whom they are subsequently administered (i.e. the donor and recipient individual are different). The donor and recipient individuals may be HLA matched to avoid GVHD and other undesirable immune effects. A suitable population of modified T cells for administration to a recipient individual may be produced by a method comprising providing an initial population of T cells obtained from a donor individual, modifying the T cells to express a CoStAR which binds specifically to cancer cells in the recipient individual, and culturing the modified T cells.

Following administration (not claimed) of the modified T cells, the recipient individual may exhibit a T cell mediated immune response against cancer cells in the recipient individual. This may have a beneficial effect on the cancer condition in the individual.

Cancer conditions may be characterised by the abnormal proliferation of malignant cancer cells and may include leukaemias, such as AML, CML, ALL and CLL, lymphomas, such as Hodgkin lymphoma, non-Hodgkin lymphoma and multiple myeloma, and solid cancers such as sarcomas, skin cancer, melanoma, bladder cancer, brain cancer, breast cancer, uterus cancer, ovary cancer, prostate cancer, lung cancer, colorectal cancer, cervical cancer, liver cancer, head and neck cancer, oesophageal cancer, pancreas cancer, renal cancer, adrenal cancer, stomach cancer, testicular cancer, cancer of the gall bladder and biliary tracts, thyroid cancer, thymus cancer, cancer of bone, and cerebral cancer, as well as cancer of unknown primary (CUP).

Cancer cells within an individual may be immunologically distinct from normal somatic cells in the individual (i.e. the cancerous tumour may be immunogenic). For example, the cancer cells may be capable of eliciting a systemic immune response in the individual against one or more antigens expressed by the cancer cells. The tumour antigens that elicit the immune response may be specific to cancer cells or may be shared by one or more normal cells in the individual.

An individual suitable for treatment as described above may be a mammal, such as a rodent (e.g. a guinea pig, a hamster, a rat, a mouse), murine (e.g. a mouse), canine (e.g. a dog), feline (e.g. a cat), equine (e.g. a horse), a primate, simian (e.g. a monkey or ape), a monkey (e.g. marmoset, baboon), an ape (e.g. gorilla, chimpanzee, orang-utan, gibbon), or a human.

In preferred arrangements, the individual is a human. In other preferred arrangements, non-human mammals, especially mammals that are conventionally used as models for demonstrating therapeutic efficacy in humans (e.g. murine, primate, porcine, canine, or rabbit animals) may be employed.

### METHOD OF TREATMENT

The term "therapeutically effective amount" refers to an amount of a CoStAR or composition comprising a CoStAR as disclosed herein, effective to "treat" a disease or disorder in an individual. In the case of cancer, the therapeutically effective amount of a CoStAR or composition comprising a CoStAR as disclosed herein can reduce the number of cancer cells; reduce the tumour size or weight; inhibit (i.e., slow to some extent and preferably stop) cancer cell infiltration into peripheral organs; inhibit (i.e., slow to some extent and preferably stop) tumour metastasis; inhibit, to some extent, tumour growth; and/or relieve to some extent one or more of the symptoms associated with the cancer. To the extent a CoStAR or composition comprising a CoStAR as disclosed herein can prevent growth and/or kill existing cancer cells, it can be cytostatic and/or cytotoxic. In some arrangements, the therapeutically effective amount is a growth inhibitory amount. In some arrangements, the therapeutically effective amount is an amount that improves progression free survival of a patient. In the case of infectious disease, such as viral infection, the therapeutically effective amount of a CoStAR or composition comprising a CoStAR as disclosed herein can reduce the number of cells infected by the pathogen; reduce the production or release of pathogen-derived antigens; inhibit (i.e., slow to some extent and preferably stop) spread of the pathogen to uninfected cells; and/or relieve to some extent one or more symptoms associated with the infection. In some arrangements, the therapeutically effective amount is an amount that extends the survival of a patient.

Cells, including T and NK cells, expressing CoStARs for use in the methods of the present disclosure may either be created ex vivo either from a patient's own peripheral blood (autologous), or in the setting of a haematopoietic stem cell transplant from donor peripheral blood (allogenic), or peripheral blood from an unconnected donor (allogenic). Alternatively, T-cells or NK cells may be derived from ex-vivo differentiation of inducible progenitor cells or embryonic progenitor cells to T-cells or NK cells. In these instances, T-cells expressing a CoStAR and, optionally, a CAR and/or TCR, are generated by introducing DNA or RNA coding for the CoStAR and, optionally, a CAR and/or TCR, by one of many means including transduction with a viral vector, transfection with DNA or RNA.

T or NK cells expressing a CoStAR of the present invention and, optionally, expressing a TCR and/or CAR may be used for the treatment of haematological cancers or solid tumours.

A method for the treatment of disease (not claimed) relates to the therapeutic use of a vector or cell, including a T or NK cell, of the invention. In this respect, the vector, or T or NK cell may be administered to a subject having an existing disease or condition in order to lessen, reduce or improve at least one symptom associated with the disease and/or to slow down, reduce or block the progression of the disease. The method may cause or promote T-cell mediated killing of cancer cells. The vector, or T or NK cell according to the present disclosure may be administered (not claimed) to a patient with one or more additional therapeutic agents. The one or more additional therapeutic agents can be co-administered to the patient. By "co-administering" is meant administering one or more additional therapeutic agents and the vector, or T or NK cell of the present invention sufficiently close in time such that the vector, or T or NK cell can enhance the effect of one or more additional therapeutic agents, or vice versa. In this regard, the vectors or cells can be administered first and the one or more additional therapeutic agents can be administered second, or vice versa. Alternatively, the vectors or cells and the one or more additional therapeutic agents can be administered simultaneously. One co-administered therapeutic agent that may be useful is IL-2, as this is currently used in existing cell therapies to boost the activity of administered cells. However, IL-2 treatment is associated with toxicity and tolerability issues.

As mentioned, for administration to a patient, the CoStAR effector cells can be allogeneic or autologous to the patient. In certain arrangements, allogeneic cells are further genetically modified, for example by gene editing, so as to minimize or prevent GVHD and/or a patient's immune response against the CoStAR cells.

The CoStAR effector cells are used to treat cancers and neoplastic diseases associated with a target antigen. Cancers and neoplastic diseases that may be treated using any of the methods described herein include tumours that are not vascularized, or not yet substantially vascularized, as well as vascularized tumours. The cancers may comprise non-solid tumours (such as hematological tumours, for example, leukemias and lymphomas) or may comprise solid tumours. Types of cancers to be treated with the CoStAR effector cells of the invention include, but are not limited to, carcinoma, blastoma, and sarcoma, and certain leukemia or lymphoid malignancies, benign and malignant tumours, and malignancies e.g.
sarcomas, carcinomas, and melanomas. Adult tumours/cancers and pediatric tumours/cancers are also included.

Hematologic cancers are cancers of the blood or bone marrow. Examples of hematological (or hematogenous) cancers include leukemias, including acute leukemias (such as acute lymphocytic leukemia, acute myelocytic leukemia, acute myelogenous leukemia and myeloblastic, promyelocytic, myelomonocytic, monocytic and erythroleukemia), chronic leukemias (such as chronic myelocytic (granulocytic) leukemia, chronic myelogenous leukemia, and chronic lymphocytic leukemia), polycythemia vera, lymphoma, Hodgkin's disease, non-Hodgkin's lymphoma (indolent and high grade forms), multiple myeloma, plasmacytoma, Waldenstrom's macroglobulinemia, heavy chain disease, myelodysplastic syndrome, hairy cell leukemia and myelodysplasia.

Solid tumours are abnormal masses of tissue that usually do not contain cysts or liquid areas. Solid tumours can be benign or malignant. Different types of solid tumours are named for the type of cells that form them (such as sarcomas, carcinomas, and lymphomas). Examples of solid tumours, such as sarcomas and carcinomas, include adrenocortical carcinoma, cholangiocarcinoma, fibrosarcoma, myxosarcoma, liposarcoma, chondrosarcoma, osteosarcoma, and other sarcomas, synovioma, mesothelioma, Ewing's tumour, leiomyosarcoma, rhabdomyosarcoma, colon carcinoma, stomach cancer, lymphoid malignancy, pancreatic cancer, breast cancer, lung cancers, ovarian cancer, prostate cancer, hepatocellular carcinoma, squamous cell carcinoma, basal cell carcinoma, adenocarcinoma, sweat gland carcinoma, thyroid cancer (e.g., medullary thyroid carcinoma and papillary thyroid carcinoma), pheochromocytomas sebaceous gland carcinoma, papillary carcinoma, papillary adenocarcinomas, medullary carcinoma, bronchogenic carcinoma, renal cell carcinoma, hepatoma, bile duct carcinoma, choriocarcinoma, Wilms' tumour, cervical cancer (e.g., cervical carcinoma and pre-invasive cervical dysplasia), colorectal cancer, cancer of the anus, anal canal, or anorectum, vaginal cancer, cancer of the vulva (e.g., squamous cell carcinoma, intraepithelial carcinoma, adenocarcinoma, and fibrosarcoma), penile cancer, oropharyngeal cancer, esophageal cancer, head cancers (e.g., squamous cell carcinoma), neck cancers (e.g., squamous cell carcinoma), testicular cancer (e.g., seminoma, teratoma, embryonal carcinoma, teratocarcinoma, choriocarcinoma, sarcoma, Leydig cell tumour, fibroma, fibroadenoma, adenomatoid tumours, and lipoma), bladder carcinoma, kidney cancer, melanoma, cancer of the uterus (e.g., endometrial carcinoma), urothelial cancers (e.g., squamous cell carcinoma, transitional cell carcinoma, adenocarcinoma, ureter cancer, and urinary bladder cancer), and CNS tumours (such as a glioma (such as brainstem glioma and mixed gliomas), glioblastoma (also known as glioblastoma multiforme) astrocytoma, CNS lymphoma, germinoma, medulloblastoma, Schwannoma craniopharyogioma, ependymoma, pinealoma, hemangioblastoma, acoustic neuroma, oligodendroglioma, menangioma, neuroblastoma, retinoblastoma and brain metastases).

When "an immunologically effective amount," "an anti-tumour effective amount," "a tumour-inhibiting effective amount," or "therapeutic amount" is indicated, the precise amount of the compositions of the present invention to be administered (not claimed) can be determined by a physician with consideration of individual differences in age, weight, tumour size, extent of infection or metastasis, and condition of the patient (subject). It can generally be stated that a pharmaceutical composition comprising the T cells described herein may be administered at a dosage of 10⁴ to 10⁹ cells/kg body weight, in some instances 10⁵ to 10⁶ cells/kg body weight, including all integer values within those ranges. T cell compositions may also be administered multiple times at these dosages. The cells can be administered by using infusion techniques that are commonly known in immunotherapy (see, e.g., Rosenberg et al., New Eng. J. of Med. 319:1676, 1988).

### Combination Therapies

A CoStAR-expressing cell described herein may be used in combination with other known agents and therapies. Administered "in combination", as used herein, means that two (or more) different treatments are delivered to the subject during the course of the subject's affliction with the disorder, e.g., the two or more treatments are delivered after the subject has been diagnosed with the disorder and before the disorder has been cured or eliminated or treatment has ceased for other reasons. In some arrangements (not claimed), the delivery of one treatment is still occurring when the delivery of the second begins, so that there is overlap in terms of administration. This is sometimes referred to herein as "simultaneous" or "concurrent delivery". In other arrangements (not claimed), the delivery of one treatment ends before the delivery of the other treatment begins. In some arrangements (not claimed) of either case, the treatment is more effective because of combined administration. For example, the second treatment is more effective, e.g., an equivalent effect is seen with less of the second treatment, or the second treatment reduces symptoms to a greater extent, than would be seen if the second treatment were administered in the absence of the first treatment, or the analogous situation is seen with the first treatment. In some arrangements (not claimed), delivery is such that the reduction in a symptom, or other parameter related to the disorder is greater than what would be observed with one treatment delivered in the absence of the other. The effect of the two treatments can be partially additive, wholly additive, or greater than additive. The delivery can be such that an effect of the first treatment delivered is still detectable when the second is delivered.

A CoStAR-expressing cell described herein and the at least one additional therapeutic agent can be administered (not claimed) simultaneously, in the same or in separate compositions, or sequentially. For sequential administration (not claimed), the CAR-expressing cell described herein can be administered first, and the additional agent can be administered second, or the order of administration can be reversed.

The CoStAR therapy and/or other therapeutic agents, procedures or modalities can be administered (not claimed) during periods of active disorder, or during a period of remission or less active disease. The CoStAR therapy can be administered (not claimed) before the other treatment, concurrently with the treatment, post-treatment, or during remission of the disorder.

When administered in combination (not claimed), the therapy and the additional agent (e.g., second or third agent), or all, can be administered in an amount or dose that is higher, lower or the same than the amount or dosage of each agent used individually, e.g., as a monotherapy. In certain arrangements (not claimed), the administered amount or dosage of the CoStAR therapy, the additional agent (e.g., second or third agent), or all, is lower (e.g., at least 20%, at least 30%, at least 40%, or at least 50%) than the amount or dosage of each agent used individually, e.g., as a monotherapy. In other arrangements (not claimed), the amount or dosage of the CoStAR therapy, the additional agent (e.g., second or third agent), or all, that results in a desired effect (e.g., treatment of cancer) is lower (e.g., at least 20%, at least 30%, at least 40%, or at least 50% lower) than the amount or dosage of each agent used individually, e.g., as a monotherapy, required to achieve the same therapeutic effect.

In further arrangements, a CoStAR-expressing cell described herein may be used in a treatment regimen (not claimed) in combination with surgery, chemotherapy, radiation, immunosuppressive agents, such as cyclosporin, azathioprine, methotrexate, mycophenolate, and FK506, antibodies, or other immunoablative agents such as CAMPATH, anti-CD3 antibodies or other antibody therapies, cytoxin, fludarabine, cyclosporin, FK506, rapamycin, mycophenolic acid, steroids, FR901228, cytokines, and irradiation, peptide vaccine, such as that described in Izumoto et al. 2008 J Neurosurg 108:963-971.

In certain instances, compounds of the present invention are combined with other therapeutic agents, such as other anti-cancer agents, anti-allergic agents, anti-nausea agents (or anti-emetics), pain relievers, cytoprotective agents, and combinations thereof.

In one arrangement, a CoStAR-expressing cell described herein can be used in combination with a chemotherapeutic agent. Exemplary chemotherapeutic agents include an anthracycline (e.g., doxorubicin (e.g., liposomal doxorubicin)), a vinca alkaloid (e.g., vinblastine, vincristine, vindesine, vinorelbine), an alkylating agent (e.g., cyclophosphamide, decarbazine, melphalan, ifosfamide, temozolomide), an immune cell antibody (e.g., alemtuzamab, gemtuzumab, rituximab, ofatumumab, tositumomab, brentuximab), an antimetabolite (including, e.g., folic acid antagonists, pyrimidine analogs, purine analogs and adenosine deaminase inhibitors (e.g., fludarabine)), an mTOR inhibitor, a TNFR glucocorticoid induced TNFR related protein (GITR) agonist, a proteasome inhibitor (e.g., aclacinomycin A, gliotoxin or bortezomib), an immunomodulator such as thalidomide or a thalidomide derivative (e.g., lenalidomide).

General Chemotherapeutic agents considered for use in combination therapies include busulfan (Myleran^{®}), busulfan injection (Busulfex^{®}), cladribine (Leustatin^{®}), cyclophosphamide (Cytoxan^{®} or Neosar^{®}), cytarabine, cytosine arabinoside (Cytosar-U^{®}), cytarabine liposome injection (DepoCyt^{®}),, daunorubicin hydrochloride (Cerubidine^{®}), daunorubicin citrate liposome injection (DaunoXome^{®}), dexamethasone, , doxorubicin hydrochloride (Adriamycin^{®}, Rubex^{®}), etoposide (Vepesid^{®}), fludarabine phosphate (Fludara^{®}), hydroxyurea (Hydrea^{®}), Idarubicin (Idamycin^{®}), mitoxantrone (Novantrone^{®}), Gemtuzumab Ozogamicin (mylotarg^{®}).

General chemotherapeutic agents considered for use in combination therapies include anastrozole (Arimidex^{®}), bicalutamide (Casodex^{®}), bleomycin sulfate (Blenoxane^{®}), busulfan (Myleran^{®}), busulfan injection (Busulfex^{®}), capecitabine (Xeloda^{®}), N4-pentoxycarbonyl-5-deoxy-5-fluorocytidine, carboplatin (Paraplatin^{®}), carmustine (BiCNU^{®}), chlorambucil (Leukeran^{®}), cisplatin (Platinol^{®}), cladribine (Leustatin^{®}), cyclophosphamide (Cytoxan^{®} or Neosar^{®}), cytarabine, cytosine arabinoside (Cytosar-U^{®}), cytarabine liposome injection (DepoCyt^{®}), dacarbazine (DTIC-Dome^{®}), dactinomycin (Actinomycin D, Cosmegan), daunorubicin hydrochloride (Cerubidine^{®}), daunorubicin citrate liposome injection (DaunoXome^{®}), dexamethasone, docetaxel (Taxotere^{®}), doxorubicin hydrochloride (Adriamycin^{®}, Rubex^{®}), etoposide (Vepesid^{®}), fludarabine phosphate (Fludara^{®}), 5-fluorouracil (Adrucil^{®}, Efudex^{®}), flutamide (Eulexin^{®}), tezacitibine, Gemcitabine (difluorodeoxycitidine), hydroxyurea (Hydrea^{®}), Idarubicin (Idamycin^{®}), ifosfamide (IFEX^{®}), irinotecan (Camptosar^{®}), L-asparaginase (ELSPAR^{®}), leucovorin calcium, melphalan (Alkeran^{®}), 6-mercaptopurine (Purinethol^{®}), methotrexate (Folex^{®}), mitoxantrone (Novantrone^{®}), mylotarg, paclitaxel (Taxol^{®}), phoenix (Yttrium90/MX-DTPA), pentostatin, polifeprosan 20 with carmustine implant (Gliadel^{®}), tamoxifen citrate (Nolvadex^{®}), teniposide (Vumon^{®}), 6-thioguanine, thiotepa, tirapazamine (Tirazone^{®}), topotecan hydrochloride for injection (Hycamptin^{®}), vinblastine (Velban^{®}), vincristine (Oncovin^{®}), and vinorelbine (Navelbine^{®}).

Treatments can be evaluated, for example, by tumour regression, tumour weight or size shrinkage, time to progression, duration of survival, progression free survival, overall response rate, duration of response, quality of life, protein expression and/or activity. Approaches to determining efficacy of the therapy can be employed, including for example, measurement of response through radiological imaging.

### EXAMPLES

### Example 1 - Production of T-cells expressing CoStAR

### Materials and Methods

Construct design - The MFE23 CoStAR consists of an MFE23 derived single chain antibody fragment nucleotide sequence with an oncostatin M1 leader sequence fused to the entire human CD28 nucleic acid sequence. The CoStAR nucleotide sequence was codon optimised and gene synthesised by Genewiz Inc. The constructs were cloned into pSF.Lenti (Oxford Genetics) via an XbaI and NheI site.

Lentiviral Production - Lentiviral production was performed using a three-plasmid packaging system (Cell Biolabs, San Diego, USA) by mixing 10 µg of each plasmid, plus 10 µg of the pSF.Lenti lentiviral plasmid containing the transgene, together in serum free
RPMI containing 50 mM CaCl₂. The mixture was added dropwise to a 50% confluent monolayer of 293T cells in 75 cm² flasks. The viral supernatants were collected at 48 and 72h post transfection, pooled and concentrated using LentiPac lentiviral supernatant concentration (GeneCopoeia, Rockville, Maryland, USA) solution according to the manufacturer's instructions. Lentiviral supernatants were concentrated 10-fold and used to directly infect primary human T-cells in the presence of 4 µg/ml polybrene (Sigma-Aldrich, Dorset, UK). Peripheral blood mononuclear cells were isolated from normal healthy donors before activation for 24 hours with T-cell activation and expansion beads (Invitrogen) according to the manufacturer's instructions before addition of lentiviral supernatants.

Cell transduction was assessed 96 hours post infection using CEA.hFc protein and anti-hFc-PE secondary, plus anti-CD34-APC or by anti-CD34-PE antibodies alone. Cells were then expanded further using x10 donor mismatched irradiated PBMC feeders at a 1:20-1:200 ratio in RPMI + 10% FCS with the addition of 1 µg/ml PHA and 200 IU/ml IL-2. After 14 days the cells were stained as previous and stored ready for assay.

Functionality assays were performed by mixing CoStAR positive or negative cells with wild-type or OKT3 engineered CEA-Positive LoVo or LS174T cells. Briefly, T-cells were mixed with LoVo cells at varying ratios in 96-well plates and IFNγ or IL-2 measured by ELISA. The remaining cells were incubated with 1:10 dilution of WST-1 reagent (Sigma, UK) for 30 min before absorbance reading at 450nm. % Cytotoxicity was determined using the following equation = 100 - ((Experimental reading - T-cells alone) / (tumour alone)) x100.

Proliferation assays were performed by first loading T-cells with 10 µM eFluor450 proliferation dye (eBioscience, UK) for 10 min at 37°C at a concentration of 1×10⁷ cells/ml before incubating the cells in 5 volumes of cold T-cell media for 5 min on ice. Cells were then washed excessively to remove unbound dye and added to cocultures containing tumour cells. Cells were removed at 2, 6 and 10 days, 1:200 dilution of DRAQ7 added and the cells analysed using a MACSQuant cytometer and MACSQuantify software.

Cell counts for proliferation assays were performed by taking cells from the wells and staining with anti-CD2 PerCP eFluor710 antibody (eBioscience, UK) for 20 min in the dark, followed by DRAQ7 staining and counts made using a MACSQuant analyser.

RESULTS - Primary human T-cells were isolated from Buffy coats obtained from the NHSBT. T-cells were isolated by Ficoll-mediated isolation and T-cell negative isolation kits (StemCell Technologies). The isolated T-cells were activated with human T-cell activation and expansion beads (Invitrogen, UK). Cells were incubated with concentrated lentiviral particles and expanded over a number of days. The lentivirus contained the DNA sequence of the MFE.CoStAR.2A.tCD34 construct (MFE23.scFv fused to full length human CD28 co-expressed with truncated human CD34 via a 2A cleavage sequence). Successfully transduced cells were further expanded using irradiated feeders as outlined in materials and methods. Donor 1 transduction was measured at 22.69% (17.15 CD34+/CoStAR+ plus 5.53% CD34-/CoStAR+), donor 2 was measured at 20.73%, and donor 3 at 13.34%. Cells were enriched for CoStAR expression using anti-CD34 antibodies to obtain T-cell populations greater than 90% CoStAR positive.

To generate a physiologically relevant in vitro model to test the impact of CoStAR on T-cell activity, the non-transduced and transduced cells were tested against the CEA+ tumour cell lines LoVo and LS174T. To enable activation of the T-cells in response to the unmatched tumour lines we engineered the tumour cells to express an anti-CD3 single chain antibody fragment anchored to the cell membrane by way of a synthetic transmembrane domain and split from the GFP marker gene using an IRES element to visualise transduced cells using flow cytometry.

Single cell clones of LoVo and LS174T were generated from bulk transfectants. Non-transduced and CoStAR transduced T-cells were mixed at varying effector:target ratios with wild-type non-transduced or OKT3-engineered LS174T or LoVo cells. After 24 hours coculture media was taken for IL-2 ELISA measurement. Activation dependent IL-2 secretion was observed from both CoStAR+ and CoStAR- T-cell populations from three donors in response to OKT3 engineered LS174T cells with only background IL-2 secretion seen from transduced and non-transduced T-cells in response to un-engineered tumour cells (Fig. 3 A-C). CoStAR enhanced IL-2 secretion towards OKT3 engineered tumour cells was found in all three donors tested. The effect was most evident at E:T ratios of 8:1 and 16:1 and at higher E:T ratios IL-2 secretion was too low to measure accurately. At lower effector to target ratios it appeared that IL-2 secretion was saturating from non-transduced cells. These observations were repeated in LoVo cells with two of the three donors tested against LS174T with similar results (Fig. 3D & E).

To determined the impact of CoStAR on T-cell expansion, transduced or non-transduced T-cells were mixed with wild-type or OKT3-GFP engineered LoVo cells the number of total cells after 3 days was counted. CoStAR enhanced survival and/or proliferation of engineered T-cells in response to LoVo-OKT3 but not wild-type LoVo cells in the presence of IL-2 (Fig. 4A) and absence of IL-2 (Fig. 4B). To further investigated this phenomenon, cell proliferation analysis was performed in T-cells from two donors using proliferation dye to count the number of cell cycles each population went through over 6 days (Fig. 4C & D). A larger proportion of CoStAR engineered cells went through 5,6 or 7 proliferation cycles over 6 days compared to non-engineered cells in response to LoVo-OKT3, whereas CoStAR transduced and non-transduced cells went through an average of approximately 2 cycles over the same duration in response to wild-type LoVo.

A variety of fusion receptors consisting of CD28 fused to an N-terminal additional costimulatory domain were generated. Costimulatory domains obtained from: CD137, CD2, CD29, CD134, CD150, CD40, GITR and the signalling domain from the IL-2 receptor γ-chain (IL2Ry) were chosen. A receptor as close to that used in previous studies of inducible costimulation was included. This receptor designated CD28(IEV) is truncated such that the C-terminal motif of CD28 is the amino acid triad 'IEV'. Sequences were generated de novo by Genewiz and cloned into a lentiviral vector under an EF1α promoter along with a CD34 marker gene separated from the fusion CoStAR by a 2A self-cleaving peptide. Primary CD8+ T-cells were isolated using EasySep beads (StemCell Technologies) and activated with anti-CD3/anti-CD28 activation/expansion Dynabeads before addition of lentiviral particles. Following a short expansion period the cells were mixed with LoVo or LoVo-OKT3 cells, with the inclusion of anti-CD107a antibodies and brefeldin and monensin, and following a 16 hour incubation were fixed and stained with antibodies to the marker gene (CD34) as well as antibodies to IL-2, IFNγ and bcl-xL. Analysis was performed using a MACSQuant analyser and MACSQuantify software. Fig. 5 shows the IL-2 response from CD34- (CoStAR non-transduced) and CD34+ (CoStAR transduced). Statistical analysis demonstrated that all receptors tested induced a significant increase in the proportion of cells producing IL-2 when harbouring the variant CoStAR receptors. Three other read outs were concurrently measured: IFNγ, a cytokine released under normal signal 1 conditions but enhanced by costimulation; CD107a, a marker of degranulation; and bcl-xL, an antiapoptotic protein upregulated by costimulation. Engagement of CoStAR enhanced all the effector functions analysed to varying degrees. CD28.CD2 and CD28.CD40 fusions receptors appeared to elicit the most robust response of all the receptors tested (See Fig. 6)

### Example 2

The effect of CD28 and CD28.CD40 based CoStARs on population based cytokine secretion was compared. Primary T-cells from three donors were transduced with either the CD28(IEV) truncated CoStAR, full length CD28 CoStAR or CD28.CD40 CoStAR (having the full length CD28 as shown inSEQ ID NO. 10, but lacking the N terminal N and K residues) or left non-transduced. T-cells were enriched for CoStAR expression using the CD34 marker gene, and following expansion cells were mixed with LoVo-OKT3 cells and IL-2 secretion analysed by ELISA (See Fig. 7). Non-transduced cells on average produced 0.80 ng/ml IL-2, with CD28(IEV) and full length CD28 CoStAR producing 4.6 and 5.0 ng/ml IL-2 respectively. However CD28.CD40 induced 29.0 ng/ml IL-2 on average across three donors thus demonstrating a clear benefit to incorporating CD40 into the basic CD28-based CoStAR.

Next the effect of CoStAR on T-cell expansion was analysed. T-cells from seven donors were transduced with either CD28 or CD28.CD40 CoStARs with either an anti-CA125 (196-14) or anti-Folate receptor (MOV-19) scFv, or an anti-Folate receptor peptide (C7) antigen binding domain. Additional cells were transduced with a CD28 CoStAR harboring an anti-CEA scFv as a mismatched control. Cells were then mixed with CA125+/Folate receptor+/CEA- cell line OvCAR3 engineered to express a membrane bound OKT3 (OvCAR-OKT3). T-cell counts were made after 7, 14 and 21 days, and fresh OvCAR-OKT3 added at days 7, and 14. Limited expansion of cells harbouring the anti-CA125 scFv was observed (mean fold expansion: CD28: 15.1; CD28.CD40: 69.1), however cells targeting Folate receptor with an scFv did expand in both the CD28 and CD28.CD40 cohorts (mean fold expansion: CD28: 186.7; CD28.CD40: 1295.0). More limited expansion was seen when the C7 peptide was used to target the Folate receptor (mean fold expansion: CD28: 71.5; CD28.CD40: 28.0). The control CEA targeting receptor demonstrated limited expansion (mean fold expansion: 28.0).

To better understand the synergy of signal 1 and signal 2 T-cells were engineered with a murine constant domain modified TCR which recognizes a CEA peptide (691-699) in the context of HLA-A*02 as well as the CD28 or CD28.CD40 CoStAR targeted towards cell surface CEA protein. As a control cells were also transduced with a CA125 specific CD28 CoStAR. The T-cells were mixed with HLA-A*02+/CEA+ H508 cells and cytokine production analysed by intracellular flow cytometry staining. Flow cytometric gating was performed using antibodies directed towards the murine TCRβ constant domain (marks the TCR engineered cells) as well as the DYKDDDDK (SEQ ID NO: 14) epitope tag (marks the CoStAR engineered cells). Thus it was possible to analyse the TCR-/CoStAR-, TCR+/CoStAR-, TCR-/CoStAR+ and TCR+/CoStAR+ cells in each coculture well. Cytokine production was then plotted in each subpopulation in either the CD4+ or CD8+ T-cells (Fig. 9). In CD4+ cells CD28.CD40 CoStAR enhanced CD137 and TNFα production above TCR stimulation alone, however the TCR response in CD4+ cells was poor due to the dependency of the TCR on CD8. In CD8+ cells there was more robust effector activity with IL-2 and CD107a in particular showing a stronger induction in the CD28.CD40 CoStAR groups. To better compare the receptors the effector activity in just the TCR+/CoStAR+ groups was plotted in CD4+ and CD8+ cells (Fig. 10). In CD4+ cells induction of CD137 was significantly enhanced by CD28.CD40 compared to either CEA or mismatched targeting CD28 CoStAR. In CD8+ cells CD137 induction was significantly increased compared to either CEA or mismatched targeting CD28 CoStAR, whereas CD107a induction was increased compared to the control CoStAR. Thus CD28.CD40 shows enhanced effector activity across a broad range of models and effector activities.

### References

Alvarez-Vallina L, Hawkins RE. Eur J Immunol. 1996 Oct;26(10):2304-9. Antigen-specific targeting of CD28-mediated T cell co-stimulation using chimeric single-chain antibody variable fragment-CD28 receptors.
Bridgeman JS, Hawkins RE, Bagley S, Blaylock M, Holland M, Gilham DE. J Immunol. 2010 Jun 15;184(12):6938-49. The optimal antigen response of chimeric antigen receptors harboring the CD3zeta transmembrane domain is dependent upon incorporation of the receptor into the endogenous TCR/CD3 complex.
Govers C, Sebestyén Z, Roszik J, van Brakel M, Berrevoets C, Szö 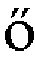 r A, Panoutsopoulou K, Broertjes M, Van T, Vereb G, Szöll 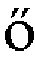 si J, Debets R. J Immunol. 2014 Nov 15;193(10):5315-26. TCRs genetically linked to CD28 and CD3ε do not mispair with endogenous TCR chains and mediate enhanced T cell persistence and anti-melanoma activity.
Grupp SA, Kalos M, Barrett D, Aplenc R, Porter DL, Rheingold SR, Teachey DT, Chew A, Hauck B, Wright JF, Milone MC, Levine BL, June CH. N Engl J Med. 2013 Apr 18;368(16): 1509-18. Chimeric antigen receptor-modified T cells for acute lymphoid leukemia.
Kochenderfer JN, Dudley ME, Kassim SH, Somerville RP, Carpenter RO, Stetler-Stevenson M, Yang JC, Phan GQ, Hughes MS, Sherry RM, Raffeld M, Feldman S, Lu L, Li YF, Ngo LT, Goy A, SA, Rosenberg SA. Science. 2006 Oct 6;314(5796): 126-9. Cancer regression in patients after transfer of genetically engineered lymphocytes.
Krause A, Guo HF, Latouche JB, Tan C, Cheung NK, Sadelain M. J Exp Med. 1998 Aug 17;188(4):619-26. Antigen-dependent CD28 signaling selectively enhances survival and proliferation in genetically modified activated human primary T lymphocytes.
Lanitis E, Poussin M, Klattenhoff AW, Song D, Sandaltzopoulos R, June CH, Powell DJ Jr., Cancer Immunol Res. 2013 Jul;1(1):43-53. Chimeric antigen receptor T Cells with dissociated signaling domains exhibit focused antitumour activity with reduced potential for toxicity in vivo.
Rapoport AP, Stadtmauer EA, Binder-Scholl GK, Goloubeva O, Vogl DT, Lacey SF, Badros AZ, Garfall A, Weiss B, Finklestein J, Kulikovskaya I, Sinha SK, Kronsberg S, Gupta M, Bond S, Melchiori L, Brewer JE, Bennett AD, Gerry AB, Pumphrey NJ, Williams D, Tayton-Martin HK, Ribeiro L, Holdich T, Yanovich S, Hardy N, Yared J, Kerr N, Philip S, Westphal S, Siegel DL, Levine BL, Jakobsen BK, Kalos M, June CH. Nat Med. 2015 Aug;21(8):914-21. NY-ESO-1-specific TCR-engineered T cells mediate sustained antigen-specific antitumour effects in myeloma.
Rosenberg SA, Yang JC, Sherry RM, Kammula US, Hughes MS, Phan GQ, Citrin DE, Restifo NP, Robbins PF, Wunderlich JR, Morton KE, Laurencot CM, Steinberg SM, White DE, Dudley ME. Clin Cancer Res. 2011 Jul 1;17(13):4550-7. Durable complete responses in heavily pretreated patients with metastatic melanoma using T-cell transfer immunotherapy.

### SEQUENCES

SEQ ID NO:1: The entire human CD28 mRNA transcript variant transcript with the open reading frame highlighted in bold.
LOCUS NM_006139 4900 bp mRNA linear PRI 07-OCT-2016
DEFINITION Homo sapiens CD28 molecule (CD28), transcript variant 1, mRNA.
ACCESSION NM_006139
ORIGIN 
1 taaagtcatc aaaacaacgt tatatcctgt gtgaaatgct gcagtcagga tgccttgtgg
61 tttgagtgcc ttgatcatgt gccctaaggg gatggtggcg gtggtggtgg ccgtggatga
121 cggagactct caggccttgg caggtgcgtc tttcagttcc cctcacactt cgggttcctc
181 ggggaggagg ggctggaacc ctagcccatc gtcaggacaa agatgctcag gctgctcttg
241 gctctcaact tattcccttc aattcaagta acaggaaaca agattttggt gaagcagtcg
301 cccatgcttg tagcgtacga caatgcggtc aaccttagct gcaagtattc ctacaatctc
361 ttctcaaggg agttccgggc atcccttcac aaaggactgg atagtgctgt ggaagtctgt
421 gttgtatatg ggaattactc ccagcagctt caggtttact caaaaacggg gttcaactgt
481 gatgggaaat tgggcaatga atcagtgaca ttctacctcc agaatttgta tgttaaccaa
541 acagatattt acttctgcaa aattgaagtt atgtatcctc ctccttacct agacaatgag
601 aagagcaatg gaaccattat ccatgtgaaa gggaaacacc tttgtccaag tcccctattt
661 cccggacctt ctaagccctt ttgggtgctg gtggtggttg gtggagtcct ggcttgctat
721 agcttgctag taacagtggc ctttattatt ttctgggtga ggagtaagag gagcaggctc
 
781 ctgcacagtg actacatgaa catgactccc cgccgccccg ggcccacccg caagcattac
841 cagccctatg ccccaccacg cgacttcgca gcctatcgct cctgacacgg acgcctatcc
901 agaagccagc cggctggcag cccccatctg ctcaatatca ctgctctgga taggaaatga
961 ccgccatctc cagccggcca cctcaggccc ctgttgggcc accaatgcca atttttctcg
1021 agtgactaga ccaaatatca agatcatttt gagactctga aatgaagtaa aagagatttc
1081 ctgtgacagg ccaagtctta cagtgccatg gcccacattc caacttacca tgtacttagt
1141 gacttgactg agaagttagg gtagaaaaca aaaagggagt ggattctggg agcctcttcc
1201 ctttctcact cacctgcaca tctcagtcaa gcaaagtgtg gtatccacag acattttagt
1261 tgcagaagaa aggctaggaa atcattcctt ttggttaaat gggtgtttaa tcttttggtt
1321 agtgggttaa acggggtaag ttagagtagg gggagggata ggaagacata tttaaaaacc
1381 attaaaacac tgtctcccac tcatgaaatg agccacgtag ttcctattta atgctgtttt
1441 cctttagttt agaaatacat agacattgtc ttttatgaat tctgatcata tttagtcatt
1501 ttgaccaaat gagggatttg gtcaaatgag ggattccctc aaagcaatat caggtaaacc
1561 aagttgcttt cctcactccc tgtcatgaga cttcagtgtt aatgttcaca atatactttc
1621 gaaagaataa aatagttctc ctacatgaag aaagaatatg tcaggaaata aggtcacttt
1681 atgtcaaaat tatttgagta ctatgggacc tggcgcagtg gctcatgctt gtaatcccag
1741 cactttggga ggccgaggtg ggcagatcac ttgagatcag gaccagcctg gtcaagatgg
1801 tgaaactccg tctgtactaa aaatacaaaa tttagcttgg cctggtggca ggcacctgta
1861 atcccagctg cccaagaggc tgaggcatga gaatcgcttg aacctggcag gcggaggttg
1921 cagtgagccg agatagtgcc acagctctcc agcctgggcg acagagtgag actccatctc
1981 aaacaacaac aacaacaaca acaacaacaa caaaccacaa aattatttga gtactgtgaa
2041 ggattatttg tctaacagtt cattccaatc agaccaggta ggagctttcc tgtttcatat
2101 gtttcagggt tgcacagttg gtctctttaa tgtcggtgtg gagatccaaa gtgggttgtg
2161 gaaagagcgt ccataggaga agtgagaata ctgtgaaaaa gggatgttag cattcattag
2221 agtatgagga tgagtcccaa gaaggttctt tggaaggagg acgaatagaa tggagtaatg
2281 aaattcttgc catgtgctga ggagatagcc agcattaggt gacaatcttc cagaagtggt
2341 caggcagaag gtgccctggt gagagctcct ttacagggac tttatgtggt ttagggctca
2401 gagctccaaa actctgggct cagctgctcc tgtaccttgg aggtccattc acatgggaaa
2461 gtattttgga atgtgtcttt tgaagagagc atcagagttc ttaagggact gggtaaggcc
2521 tgaccctgaa atgaccatgg atatttttct acctacagtt tgagtcaact agaatatgcc
2581 tggggacctt gaagaatggc ccttcagtgg ccctcaccat ttgttcatgc ttcagttaat
2641 tcaggtgttg aaggagctta ggttttagag gcacgtagac ttggttcaag tctcgttagt
2701 agttgaatag cctcaggcaa gtcactgccc acctaagatg atggttcttc aactataaaa
2761 tggagataat ggttacaaat gtctcttcct atagtataat ctccataagg gcatggccca
 
2821 agtctgtctt tgactctgcc tatccctgac atttagtagc atgcccgaca tacaatgtta
2881 gctattggta ttattgccat atagataaat tatgtataaa aattaaactg ggcaatagcc
2941 taagaagggg ggaatattgt aacacaaatt taaacccact acgcagggat gaggtgctat
3001 aatatgagga ccttttaact tccatcattt tcctgtttct tgaaatagtt tatcttgtaa
3061 tgaaatataa ggcacctccc acttttatgt atagaaagag gtcttttaat tttttttaa
3121 tgtgagaagg aagggaggag taggaatctt gagattccag atcgaaaata ctgtactttg
3181 gttgattttt aagtgggctt ccattccatg gatttaatca gtcccaagaa gatcaaactc
3241 agcagtactt gggtgctgaa gaactgttgg atttaccctg gcacgtgtgc cacttgccag
3301 cttcttgggc acacagagtt cttcaatcca agttatcaga ttgtatttga aaatgacaga
3361 gctggagagt tttttgaaat ggcagtggca aataaataaa tacttttttt taaatggaaa
3421 gacttgatct atggtaataa atgattttgt tttctgactg gaaaaatagg cctactaaag
3481 atgaatcaca cttgagatgt ttcttactca ctctgcacag aaacaaagaa gaaatgttat
3541 acagggaagt ccgttttcac tattagtatg aaccaagaaa tggttcaaaa acagtggtag
3601 gagcaatgct ttcatagttt cagatatggt agttatgaag aaaacaatgt catttgctgc
3661 tattattgta agagtcttat aattaatggt actcctataa tttttgattg tgagctcacc
3721 tatttgggtt aagcatgcca atttaaagag accaagtgta tgtacattat gttctacata
3781 ttcagtgata aaattactaa actactatat gtctgcttta aatttgtact ttaatattgt
3841 cttttggtat taagaaagat atgctttcag aatagatatg cttcgctttg gcaaggaatt
3901 tggatagaac ttgctattta aaagaggtgt ggggtaaatc cttgtataaa tctccagttt
3961 agcctttttt gaaaaagcta gactttcaaa tactaatttc acttcaagca gggtacgttt
4021 ctggtttgtt tgcttgactt cagtcacaat ttcttatcag accaatggct gacctctttg
4081 agatgtcagg ctaggcttac ctatgtgttc tgtgtcatgt gaatgctgag aagtttgaca
4141 gagatccaac ttcagccttg accccatcag tccctcgggt taactaactg agccaccggt
4201 cctcatggct attttaatga gggtattgat ggttaaatgc atgtctgatc ccttatccca
4261 gccatttgca ctgccagctg ggaactatac cagacctgga tactgatccc aaagtgttaa
4321 attcaactac atgctggaga ttagagatgg tgccaataaa ggacccagaa ccaggatctt
4381 gattgctata gacttattaa taatccaggt caaagagagt gacacacact ctctcaagac
4441 ctggggtgag ggagtctgtg ttatctgcaa ggccatttga ggctcagaaa gtctctcttt
4501 cctatagata tatgcatact ttctgacata taggaatgta tcaggaatac tcaaccatca
4561 caggcatgtt cctacctcag ggcctttaca tgtcctgttt actctgtcta gaatgtcctt
4621 ctgtagatga cctggcttgc ctcgtcaccc ttcaggtcct tgctcaagtg tcatcttctc
4681 ccctagttaa actaccccac accctgtctg ctttccttgc ttatttttct ccatagcatt
4741 ttaccatctc ttacattaga catttttctt atttatttgt agtttataag cttcatgagg
4801 caagtaactt tgctttgttt cttgctgtat ctccagtgcc cagagcagtg cctggtatat
 4861 aataaatatt tattgactga gtgaaaaaaa aaaaaaaaaa//
SEQ ID NO:2: The human CD28 protein sequence with the:
   I. signal peptide highlighted in bold (this may be deleted from the sequence when used according to the invention); and
   II. transmembrane region underlined; and
   III. extracellular region lies between the signal peptide and transmembrane region and the cytoplasmic regions follows the transmembrane region; and
   IV. boxed region represents the first 5 amino acids of the mature protein which may be present or absent or mutated in any aspect of this invention. Also, 1, 2, 3, 4, or 5 of these residues (i.e. N, K, I, L or V) may be deleted from the sequence when used according to the invention. 
MLRLLLALNLFPSIQVTGNKILVKQSPMLVAYDNAVNLSCKYSYNLFSREFRASLH
KGLDSAVEVCVVYGNYSQQLQVYSKTGFNCDGKLGNESVTFYLQNLYVNQTDIYF
CKIEVMYPPPYLDNEKSNGTIIHVKGKHLCPSPLFPGPSKPFWVLVVVGGVLACYSLL
VTVAFIIFWVRSKRSRLLHSDYMNMTPRRPGPTRKHYQPYAPPRDFAAYRS
SEQ ID NOs:3 to 12 below are exemplar sequences of CoStAR signalling domains which may be fused to the human CD28 protein at the boxed region of SEQ ID NO:2 (which may or may not be mutated but for diagrammatic simplicity has been left in its original form) with the:
   I. double underlined region represents the CD28 derived region; and
   II. underlined region is a second costimulatory receptor derived region; and
   III. sequence in italics is a third costimulatory receptor derived region.
Note that the boxed region represents the first 5 amino acids of the mature CD28 protein which may be present or absent or mutated in any aspect of this invention. Also, 1, 2, 3, 4, o5 5 of these resideues (i.e N, K, I, L or V) may be deleted from the sequence when used according to the invention.
SEQ ID NO:3: Truncated Cytoplasmic domain CD28 Variant 
NKILVKQSPMLVAYDNAVNLSCKYSYNLFSREFRASLHKGLDSAVEVCVVYGNYSQ
QLQVYSKTGFNCDGKLGNESVTFYLQNLYVNQTDIYFCKIEVMYPPPYLDNEKSNG
TIIHVKGKHLCPSPLFPGPSKPFWVLVVVGGVLACYSLLVTVAFIIFWVRSKR
SEQ ID NO:4: CD28.CD137 fusion 
NKILVKQSPMLVAYDNAVNLSCKYSYNLFSREFRASLHKGLDSAVEVCVVYGNYSQ
QLQVYSKTGFNCDGKLGNESVTFYLQNLYVNQTDIYFCKIEVMYPPPYLDNEKSNG
TIIHVKGKHLCPSPLFPGPSKPFWVLVVVGGVLACYSLLVTVAFIIFWVRSKRSRLLH
SDYMNMTPRRPGPTRKHYQPYAPPRDFAAYRS/RFSVVKRGRKKLLYIFKQPFMRPV
QTTQEEDGCSCRFPEEEEGGCE
SEQ ID NO:5: CD28.CD134 fusion 
NKILVKQSPMLVAYDNAVNLSCKYSYNLFSREFRASLHKGLDSAVEVCVVYGNYSQ
QLQVYSKTGFNCDGKLGNESVTFYLQNLYVNQTDIYFCKIEVMYPPPYLDNEKSNG
TIIHVKGKHLCPSPLFPGPSKPFWVLVVVGGVLACYSLLVTVAFIIFWVRSKRSRLLH
SDYMNMTPRRPGPTRKHYQPYAPPRDFAAYRS/RRDQRLPPDAHKPPGGGSFRTPIQ
EEQADAHSTLAKI
SEQ ID NO:6: CD28.CD2 fusion 
NKILVKQSPMLVAYDNAVNLSCKYSYNLFSREFRASLHKGLDSAVEVCVVYGNYSQ
QLQVYSKTGFNCDGKLGNESVTFYLQNLYVNQTDIYFCKIEVMYPPPYLDNEKSNG
TIIHVKGKHLCPSPLFPGPSKPFWVLVVVGGVLACYSLLVTVAFIIFWVRSKRSRLLH
SDYMNMTPRRPGPTRKHYQPYAPPRDFAAYRS/KRKKQRSRRNDEELETRAHRVAT
EERGRKPHQIPASTPQNPATSQHPPPPPGHRSQAPSHRPPPPGHRVQHQPQKRPPAPS
GTQVHQQKGPPLPRPRVQPKPPHGAAENSLSPSSN
SEQ ID NO:7: CD28.CD29 fusion 
NKILVKQSPMLVAYDNAVNLSCKYSYNLFSREFRASLHKGLDSAVEVCVVYGNYSQ
QLQVYSKTGFNCDGKLGNESVTFYLQNLYVNQTDIYFCKIEVMYPPPYLDNEKSNG
TIIHVKGKHLCPSPLFPGPSKPFWVLVVVGGVLACYSLLVTVAFIIFWVRSKRSRLLH
SDYMNMTPRRPGPTRKHYQPYAPPRDFAAYRS/KLLMIIHDRREFAKFEKEKMNAK
WDTGENPIYKSAVTTVVNPKYEGK
SEQ ID NO:8: CD28.GITR fusion 
NKILVKQSPMLVAYDNAVNLSCKYSYNLFSREFRASLHKGLDSAVEVCVVYGNYSQ
QLQVYSKTGFNCDGKLGNESVTFYLQNLYVNQTDIYFCKIEVMYPPPYLDNEKSNG
TIIHVKGKHLCPSPLFPGPSKPFWVLVVVGGVLACYSLLVTVAFIIFWVRSKRSRLLH
SDYMNMTPRRPGPTRKHYQPYAPPRDFAAYRS/QLGLHIWQLRSQCMWPRETQLLL
EVPPSTEDARSCQFPEEERGERSAEEKGRLGDLWV
SEQ ID NO:9: CD28.IL2Rγ fusion 
NKILVKQSPMLVAYDNAVNLSCKYSYNLFSREFRASLHKGLDSAVEVCVVYGNYSQ
QLQVYSKTGFNCDGKLGNESVTFYLQNLYVNQTDIYFCKIEVMYPPPYLDNEKSNG
TIIHVKGKHLCPSPLFPGPSKPFWVLVVVGGVLACYSLLVTVAFIIFWVRSKRSRLLH
SDYMNMTPRRPGPTRKHYQPYAPPRDFAAYRS/ERTMPRIPTLKNLEDLVTEYHGNF
SAWSGVSKGLAESLQPDYSERLCLVSEIPPKGGALGEGPGASPCNQHSPYWAPPCYT
LKPET
SEQ ID NO:10: CD28.CD40 fusion 
NKILVKQSPMLVAYDNAVNLSCKYSYNLFSREFRASLHKGLDSAVEVCVVYGNYSQ
QLQVYSKTGFNCDGKLGNESVTFYLQNLYVNQTDIYFCKIEVMYPPPYLDNEKSNG
TIIHVKGKHLCPSPLFPGPSKPFWVLVVVGGVLACYSLLVTVAFIIFWVRSKRSRLLH
SDYMNMTPRRPGPTRKHYQPYAPPRDFAAYRS/KKVAKKPTNKAPHPKQEPQEINFP
DDLPGSNTAAPVQETLHGCQPVTQEDGKESRISVQERQ
SEQ ID NO:11: CD28.CD150 fusion 
NKILVKQSPMLVAYDNAVNLSCKYSYNLFSREFRASLHKGLDSAVEVCVVYGNYSQ
QLQVYSKTGFNCDGKLGNESVTFYLQNLYVNQTDIYFCKIEVMYPPPYLDNEKSNG
TIIHVKGKHLCPSPLFPGPSKPFWVLVVVGGVLACYSLLVTVAFIIFWVRSKRSRLLH
SDYMNMTPRRPGPTRKHYQPYAPPRDFAAYRS/RRRGKTNHYQTTVEKKSLTIYAQ
VQKPGPLQKKLDSFPAQDPCTTIYVAATEPVPESVQETNSITVYASVTLPES
SEQ ID NO:12: CD28.CD2.CD40 fusion 
NKILVKQSPMLVAYDNAVNLSCKYSYNLFSREFRASLHKGLDSAVEVCVVYGNYSQ
QLQVYSKTGFNCDGKLGNESVTFYLQNLYVNQTDIYFCKIEVMYPPPYLDNEKSNG
TIIHVKGKHLCPSPLFPGPSKPFWVLVVVGGVLACYSLLVTVAFIIFWVRSKRSRLLH
SDYMNMTPRRPGPTRKHYQPYAPPRDFAAYRS/KRKKQRSRRNDEELETRAHRVAT
EERGRKPHQIPASTPQNPATSQHPPPPPGHRSQAPSHRPPPPGHRVQHQPQKRPPAPS
 
GTQVHQQKGPPLPRPRVQPKPPHGAAENSLPSSNKKVAKKPTNKAPHPKQEPQEINF
PDDLPGSNTAAPVQETLHGCQPVTQEDGKESRISVQERQ
SEQ ID NO:13: Truncated Extracellular domain CD28(IEV) Variant

NKILVKQSPMLVAYDNAVNLSCKYSYNLFSREFRASLHKGLDSAVEVCVVYGNYSQ
QLQVYSKTGFNCDGKLGNESVTFYLQNLYVNQTDIYFCKIEV

## Claims

1. A chimeric costimulatory receptor (CoStAR) comprising (i) a disease- or tumour-associated antigen binding domain; and (ii) an extracellular ligand-binding segment of a first costimulatory receptor protein, and (iii) a first intracellular segment comprising an intracellular signaling domain of the first costimulatory receptor protein, and (iv) a second intracellular segment comprising an intracellular signaling domain of a second receptor protein;
wherein the first costimulatory receptor protein is CD28; and wherein the second receptor protein is CD40,
wherein the disease- or tumour-associated antigen binding domain is attached to the extracellular ligand-binding segment of the first costimulatory receptor protein, and
wherein the disease- or tumour-associated antigen binding domain is a cancer- or tumour-associated antigen binding domain.

2. A CoStAR as claimed in claim 1, wherein the chimeric costimulatory receptor comprises SEQ ID NO:10.

3. A CoStAR as claimed in claim 1 or 2, wherein said chimeric costimulatory receptor includes the entire mature CD28 costimulatory receptor protein lacking the signal peptide and up to 5 amino acids at the N-terminus of the mature costimulatory receptor protein.

4. A CoStAR as claimed in any preceding claim wherein said chimeric costimulatory receptor includes the entire mature CD28 costimulatory receptor protein lacking the signal peptide and 1 or 2 amino acids at the N-terminus of the mature costimulatory receptor protein.

5. A CoStAR as claimed in any preceding claim, wherein the extracellular ligand-binding segment of CD28 comprises a truncation at amino acids IEV of SEQ ID NO: 2.

6. The CoStAR of any preceding claim wherein said antigen binding domain is selected from:
I. A single chain antibody fragment which binds a tumour associated antigen including, but not limited to, carcinoembryonic antigen (CEA), 5T4, mellanotransferrin (CD228), Her2, EGFR, GPC3, melanoma-associated chondroitin sulphate proteoglycan (MCSP/CSPG4), CD71, SM5-1, folate receptor or CA125; or
II. A single chain antibody fragment which binds a tumour specific peptide (p)-major histocompatability (MHC) complex; or
III. A tumour specific pMHC complex antigen specific single chain T-cell receptor (scTCR); or
IV. A natural antigen binding polypeptide such as, but not limited to, transferrin; or
V. A domain which binds an antibody (e.g. Fc binding domains such as, but not limited to, CD16, CD32 or CD64).

7. A fusion protein which comprises a CoStAR according to any preceding claim fused to a marker gene protein (e.g. DYKDDDDK (SEQ ID NO:14) epitope tag, CD34, CD19 etc), chimeric antigen receptor (CAR), a T cell receptor (TCR) or another receptor of immunotherapeutic use for adoptive cell therapy optionally which comprises a self-cleaving peptide between the CoStAR and the protein of interest.

8. A nucleic acid sequence which encodes a fusion protein comprising SEQ ID NO: 10 or a variant thereof having at least 80% sequence identity at the protein level, wherein the fusion protein is a CoStAR.

9. A vector which comprises a nucleic acid sequence according to claim 8 optionally which also comprises a sequence that encodes a chimeric antigen receptor, a T-cell receptor or another receptor of immunotherapeutic use for adoptive cell therapy, such that when the vector is used to transduce a target cell, the target cell co-expresses a CoStAR according to any of claims 1-6 and a chimeric antigen receptor, T-cell receptor or another receptor of immunotherapeutic interest.

10. A cell which expresses a CoStAR according to any of claims 1-6.

11. A cell according to claim 10 which co-expresses the CoStAR of any of claims 1-6 and a POI at the cell surface.

12. A cell which comprises a nucleic acid sequence according to claim 8.

13. A cell according to any of claims 10-12, which is a T cell.

14. A method for making a cell according to any of claims 10-13 which comprises the step of transducing or transfecting a cell with a vector according to claim 9.

15. A method for selecting cells expressing a POI which comprises the following steps:
I. detecting expression of the POI epitope on the surface of cells transfected or transduced with a vector according to claim 9; and
II. selecting cells which are identified as expressing the POI epitope.

16. A method for preparing a purified population of cells enriched for cells expressing a POI which comprises the step of selecting cells expressing a POI from a population of cells using a method according to claim 15.

17. A method according to claim 16, which comprises the ex vivo transduction or transfection of a population of cells isolated from a tissue or cell donor with:
a vector according to claim 9; and
selecting cells expressing the POI from the transduced/transfected population of cells by a method according to claim 15.

18. The cell of claim 10 for use as a medicament.

19. The cell of claim 10 for use in treating cancer and/or adoptive cell transfer.

20. The CoStAR of claim 6, further comprising:
(i) a signalling domain comprising or consisting of a full length human CD28 of SEQ ID NO:2 or a variant thereof having at least 80% sequence identity at the protein level wherein said full length human CD28 does not comprise the signal peptide and optionally lacks up to 5 amino acids at the N-terminal of the mature receptor protein once its signal peptide has been cleaved; and
(ii) the intracellular domain from human CD40 as shown in SEQ ID NO: 10 or a variant thereof having at least 80% or 90% sequence identity at the protein level, wherein the intracellular domain from human CD40 or a variant thereof has functional activity as a CD40 intracellular signalling domain.

21. The CoStAR of claim 6, wherein the folate receptor is folate receptor alpha.

## Patentansprüche

1. Ein chimärer costimulatorischer Rezeptor (CoStAR), der Folgendes beinhaltet: (i) eine krankheits- oder tumorassoziierte antigenbindende Domäne; und (ii) ein extrazelluläres ligandenbindendes Segment eines ersten costimulatorischen Rezeptorproteins und (iii) ein erstes intrazelluläres Segment, das eine intrazelluläre Signalisierungsdomäne des ersten costimulatorischen Rezeptorproteins beinhaltet, und (iv) ein zweites intrazelluläres Segment, das eine intrazelluläre Signalisierungsdomäne eines zweiten Rezeptorproteins beinhaltet;
wobei das erste costimulatorische Rezeptorprotein CD28 ist; und wobei das zweite Rezeptorprotein CD40 ist,
wobei die krankheits- oder tumorassoziierte antigenbindende Domäne an dem extrazellulären ligandenbindenden Segment des ersten costimulatorischen Rezeptorproteins gebunden ist, und
wobei die krankheits- oder tumorassoziierte antigenbindende Domäne eine krebs- oder tumorassoziierte antigenbindende Domäne ist.

2. CoStAR gemäß Anspruch 1, wobei der chimäre costimulatorische Rezeptor SEQ ID NO: 10 beinhaltet.

3. CoStAR gemäß Anspruch 1 oder 2, wobei der chimäre costimulatorische Rezeptor das gesamte reife costimulatorische CD28-Rezeptorprotein umfasst, dem das Signalpeptid und bis zu 5 Aminosäuren am N-Terminus des reifen costimulatorischen Rezeptorproteins fehlen.

4. CoStAR gemäß einem der vorhergehenden Ansprüche, wobei der chimäre costimulatorische Rezeptor das gesamte reife costimulatorische CD28-Rezeptorprotein umfasst, dem das Signalpeptid und 1 oder 2 Aminosäuren am N-Terminus des reifen costimulatorischen Rezeptorproteins fehlen.

5. CoStAR gemäß einem der vorhergehenden Ansprüche, wobei das extrazelluläre ligandenbindende Segment von CD28 eine Trunkierung an Aminosäuren IEV von SEQ ID NO: 2 beinhaltet.

6. CoStAR gemäß einem der vorhergehenden Ansprüche, wobei die antigenbindende Domäne aus Folgendem ausgewählt ist:
I. einem Einzelketten-Antikörperfragment, das ein tumorassoziiertes Antigen bindet, umfassend, aber nicht beschränkt auf, karzinoembryonales Antigen (CEA), 5T4, Mellanotransferrin (CD228), Her2, EGFR, GPC3, melanomassoziiertes Chondroitinsulfat-Proteoglycan (MCSP/CSPG4), CD71, SM5-1, Folatrezeptor oder CA125; oder
II. einem Einzelketten-Antikörperfragment, das einen tumorspezifischen Peptid-(p)-Haupthistokompatibilitäts(MHC)-Komplex bindet; oder
III. einem tumorspezifischen, pMHC-Komplex-antigenspezifischen Einzelketten-T-Zell-Rezeptor (scTCR); oder
IV. einem natürlichen antigenbindenden Polypeptid wie, aber nicht beschränkt auf, Transferrin; oder
V. einer Domäne, die einen Antikörper bindet (z. B. Fc-bindende Domänen wie, aber nicht beschränkt auf, CD16, CD32 oder CD64).

7. Ein Fusionsprotein, das einen CoStAR gemäß einem der vorhergehenden Ansprüche beinhaltet, der an ein Markergenprotein (z. B. DYKDDDDK(SEQ ID NO: 14)-Epitoptag, CD34, CD19 usw.), einen chimären Antigenrezeptor (CAR), einen T-Zell-Rezeptor (TCR) oder einen anderen Rezeptor von immuntherapeutischem Nutzen zur adoptiven Zelltherapie fusioniert ist, das wahlweise ein selbstspaltendes Peptid zwischen dem CoStAR und dem Protein von Interesse beinhaltet.

8. Eine Nukleinsäuresequenz, die ein Fusionsprotein codiert, beinhaltend SEQ ID NO: 10 oder eine Variante davon mit mindestens 80 % Sequenzidentität auf der Proteinebene, wobei das Fusionsprotein ein CoStAR ist.

9. Ein Vektor, der eine Nukleinsäuresequenz gemäß Anspruch 8 beinhaltet, der wahlweise auch eine Sequenz beinhaltet, die einen chimären Antigenrezeptor, einen T-Zell-Rezeptor oder einen anderen Rezeptor von immuntherapeutischem Nutzen zur adoptiven Zelltherapie derart codiert, dass, wenn der Vektor verwendet wird, um eine Zielzelle zu transduzieren, die Zielzelle einen CoStAR gemäß einem der Ansprüche 1-6 und einen chimären Antigenrezeptor, T-Zell-Rezeptor oder einen anderen Rezeptor von immuntherapeutischem Interesse coexprimiert.

10. Eine Zelle, die einen CoStAR gemäß einem der Ansprüche 1-6 exprimiert.

11. Zelle gemäß Anspruch 10, die den CoStAR gemäß einem der Ansprüche 1-6 und ein POI auf der Zelloberfläche exprimiert.

12. Eine Zelle, die eine Nukleinsäuresequenz gemäß Anspruch 8 beinhaltet.

13. Zelle gemäß einem der Ansprüche 10-12, die eine T-Zelle ist.

14. Ein Verfahren zum Herstellen einer Zelle gemäß einem der Ansprüche 10-13, das den Schritt des Transduzierens oder Transfizierens einer Zelle mit einem Vektor gemäß Anspruch 9 beinhaltet.

15. Ein Verfahren zum Auswählen von Zellen, die ein POI exprimieren, das die folgenden Schritte beinhaltet:
I. Detektieren von Expression des POI-Epitops auf der Oberfläche von mit einem Vektor gemäß Anspruch 9 transfizierten oder transduzierten Zellen; und
II. Auswählen von Zellen, die als das POI-Epitop exprimierend identifiziert werden.

16. Ein Verfahren zum Zubereiten einer gereinigten Population von Zellen, die mit Zellen angereichert ist, die ein POI exprimieren, das den Schritt des Auswählens von Zellen, die ein POI exprimieren, aus einer Population von Zellen unter Verwendung eines Verfahrens gemäß Anspruch 15 beinhaltet.

17. Verfahren gemäß Anspruch 16, das die ex-vivo-Transduktion oder-Transfektion einer Population von Zellen beinhaltet, die aus einem Gewebe oder von einem Zelldonor isoliert wurden, mit:
einem Vektor gemäß Anspruch 9; und
Auswählen von Zellen, die das POI exprimieren, aus der transduzierten/transfizierten Population von Zellen durch ein Verfahren gemäß Anspruch 15.

18. Zelle gemäß Anspruch 10 zur Nutzung als ein Medikament.

19. Zelle gemäß Anspruch 10 zur Nutzung beim Behandeln von Krebs und/oder bei adoptivem Zelltransfer.

20. CoStAR gemäß Anspruch 6, ferner beinhaltend:
(i) eine Signalisierungsdomäne, beinhaltend oder bestehend aus einem humanen CD28 voller Länge von SEQ ID NO: 2 oder einer Variante davon mit mindestens 80 % Sequenzidentität auf Proteinebene, wobei das humane CD28 voller Länge das Signalpeptid nicht beinhaltet und dem wahlweise bis zu 5 Aminosäuren am N-Terminal des reifen Rezeptorproteins fehlen, sobald sein Signalpeptid abgespalten wurde; und
(ii) die intrazelluläre Domäne aus humanem CD40 wie gezeigt in SEQ ID NO: 10 oder eine Variante davon mit mindestens 80 % oder 90 % Sequenzidentität auf Proteinebene, wobei die intrazelluläre Domäne aus humanem CD40 oder eine Variante davon funktionale Aktivität wie eine intrazelluläre CD40-Signalisierungsdomäne aufweist.

21. CoStAR gemäß Anspruch 6, wobei der Folatrezeptor Folatrezeptor Alpha ist.

## Revendications

1. Un récepteur de costimulation chimérique (CoStAR) comprenant (i) un domaine de liaison à un antigène associé à une maladie ou une tumeur ; et (ii) un segment extracellulaire de liaison à un ligand d'une première protéine de récepteur de costimulation, et (iii) un premier segment intracellulaire comprenant un domaine de signalisation intracellulaire de la première protéine de récepteur de costimulation, et (iv) un deuxième segment intracellulaire comprenant un domaine de signalisation intracellulaire d'une deuxième protéine de récepteur ;
où la première protéine de récepteur de costimulation est CD28 ; et où la deuxième protéine de récepteur est CD40,
où le domaine de liaison à un antigène associé à une maladie ou une tumeur est fixé au segment extracellulaire de liaison à un ligand de la première protéine de récepteur de costimulation, et
où le domaine de liaison à un antigène associé à une maladie ou une tumeur est un domaine de liaison à un antigène associé à un cancer ou une tumeur.

2. Un CoStAR tel que revendiqué dans la revendication 1, le récepteur de costimulation chimérique comprenant la SEQ ID NO : 10.

3. Un CoStAR tel que revendiqué dans la revendication 1 ou la revendication 2, ledit récepteur de costimulation chimérique incluant la protéine de récepteur de costimulation CD28 mature entière dépourvue du peptide signal et de jusqu'à 5 acides aminés à l'extrémité N-terminale de la protéine de récepteur de costimulation mature.

4. Un CoStAR tel que revendiqué dans n'importe quelle revendication précédente, ledit récepteur de costimulation chimérique incluant la protéine de récepteur de costimulation CD28 mature entière dépourvue du peptide signal et de 1 ou 2 acides aminés à l'extrémité N-terminale de la protéine de récepteur de costimulation mature.

5. Un CoStAR tel que revendiqué dans n'importe quelle revendication précédente, où le segment extracellulaire de liaison à un ligand de CD28 comprend une troncature au niveau des acides aminés IEV de la SEQ ID NO : 2.

6. Le CoStAR de n'importe quelle revendication précédente où le domaine de liaison à un antigène est sélectionné parmi :
I. un fragment d'anticorps à une seule chaîne qui se lie à un antigène associé à une tumeur incluant, entre autres, l'antigène carcinoembryonnaire (CEA), le 5T4, la mélanotransferrine (CD228), l'Her2, l'EGFR, le GPC3, le protéoglycane de sulfate de chondroïtine associé à un mélanome (MCSP/CSPG4), le CD71, le SM5-1, le récepteur des folates ou le CA125 ; ou
II. un fragment d'anticorps à une seule chaîne qui se lie à un complexe majeur d'histocompatibilité (MHC)-peptide (p) spécifique d'une tumeur ; ou
III. un récepteur des lymphocytes T à une seule chaîne spécifique d'un antigène de complexe pMHC spécifique d'une tumeur ; ou
IV. un polypeptide de liaison à un antigène naturel tel que, entre autres, la transferrine ; ou
V. un domaine qui se lie à un anticorps (p. ex. des domaines de liaison de Fc tels que, entre autres, le CD16, le CD32 ou le CD64).

7. Une protéine de fusion qui comprend un CoStAR selon n'importe quelle revendication précédente fusionné à une protéine de gène marqueur (p. ex. l'étiquette épitope DYKDDDDK (SEQ ID NO : 14), le CD34, le CD19, etc.), un récepteur antigénique chimérique (CAR), un récepteur des lymphocytes T (TCR) ou un autre récepteur à usage immunothérapeutique pour une thérapie cellulaire adoptive qui comprend facultativement un peptide auto-clivant entre le CoStAR et la protéine d'intérêt.

8. Une séquence d'acide nucléique qui code pour une protéine de fusion comprenant la SEQ ID NO : 10 ou une variante de celle-ci ayant au moins 80 % d'identité de séquence au niveau protéique, où la protéine de fusion est un CoStAR.

9. Un vecteur qui comprend une séquence d'acide nucléique selon la revendication 8 qui comprend facultativement également une séquence qui code pour un récepteur antigénique chimérique, un récepteur des lymphocytes T ou un autre récepteur à usage immunothérapeutique pour une thérapie cellulaire adoptive, de telle sorte que lorsque le vecteur est utilisé pour transduire une cellule cible, la cellule cible coexprime un CoStAR selon n'importe lesquelles des revendications 1 à 6 et un récepteur antigénique chimérique, un récepteur des lymphocytes T ou un autre récepteur d'intérêt immunothérapeutique.

10. Une cellule qui exprime un CoStAR selon n'importe lesquelles des revendications 1 à 6.

11. Une cellule selon la revendication 10 qui coexprime le CoStAR de n'importe lesquelles des revendications 1 à 6 et une protéine d'intérêt (POI) à la surface cellulaire.

12. Une cellule qui comprend une séquence d'acide nucléique selon la revendication 8.

13. Une cellule selon n'importe lesquelles des revendications 10 à 12, qui est un lymphocyte T.

14. Une méthode destinée à fabriquer une cellule selon n'importe lesquelles des revendications 10 à 13, qui comprend l'étape consistant à transduire ou transfecter une cellule avec un vecteur selon la revendication 9.

15. Une méthode destinée à sélectionner des cellules exprimant une POI qui comprend les étapes suivantes consistant à :
I. détecter l'expression de l'épitope de la POI à la surface de cellules transfectées ou transduites avec un vecteur selon la revendication 9 ; et
II. sélectionner des cellules qui sont identifiées comme exprimant l'épitope de la POI.

16. Une méthode destinée à préparer une population purifiée de cellules enrichies en cellules exprimant une POI qui comprend l'étape consistant à sélectionner des cellules exprimant une POI à partir d'une population de cellules à l'aide de la méthode selon la revendication 15.

17. Une méthode selon la revendication 16, qui comprend la transduction ou transfection ex vivo d'une population de cellules isolées provenant d'un donneur de tissus ou de cellules avec :
un vecteur selon la revendication 9 ; et
le fait de sélectionner des cellules exprimant la POI à partir de la population de cellules transduites/transfectées par une méthode selon la revendication 15.

18. La cellule de la revendication 10 destinée à une utilisation en tant que médicament.

19. La cellule de la revendication 10 destinée à une utilisation dans le traitement du cancer et/ou le transfert adoptif de cellules.

20. Le CoStAR de la revendication 6, comprenant en outre :
(i) un domaine de signalisation comprenant ou consistant en un CD28 humain pleine longueur de SEQ ID NO : 2 ou d'une variante de celle-ci ayant au moins 80 % d'identité de séquence au niveau protéique, ledit CD28 humain pleine longueur ne comprenant pas le peptide signal et facultativement étant dépourvu de jusqu'à 5 acides aminés à l'extrémité N-terminale de la protéine de récepteur mature une fois que son peptide signal a été clivé ; et
(ii) le domaine intracellulaire provenant du CD40 humain tel que présenté dans la SEQ ID NO : 10 ou une variante de celle-ci ayant au moins 80 % ou 90 % d'identité de séquence au niveau protéique, le domaine intracellulaire provenant du CD40 humain ou d'une variante de celui-ci ayant une activité fonctionnelle en tant que domaine de signalisation intracellulaire de CD40.

21. Le CoStAR de la revendication 6, où le récepteur des folates est le récepteur alpha des folates.
